# EUROPEAN PATENT SPECIFICATION

(11) **EP 1 809 738 B1**
(45) Date of publication and mention of the grant of the patent: **05.03.2014**
(21) Application number: 05821237.4
(22) Date of filing: 31.10.2005
(51) Int. Cl.: C12N 5/0783

(54) **COMPOSITIONS AND METHODS FOR TREATING HYPERPROLIFERATIVE DISORDERS**
VERBINDUNGEN UND VERFAHREN ZUR BEHANDLUNG VON HYPERPROLIFERATIVEN STÖRUNGEN
COMPOSITIONS ET PROCEDES POUR TRAITER DES ETATS HYPERPROLIFERATIFS

(30) Priority: 02.11.2004 US 624803 P
(43) Date of publication of application: 25.07.2007
(73) Proprietor: THE GOVERNMENT OF THE UNITED STATES OF AMERICA, as represented by the Secretary, Department of Health and Human Services, Rockville, MD 20852-3804 (US)
(72) Inventor: CHILDS, Richard, William, Wyatt, Rockville, MA 20850 (US); IGARASHI, Takehito, Tokyo 171-0031 (JP); WYNBERG, Jason, Benjamin, Oak Park, MI 48237 (US)
(74) Representative: Goodfellow, Hugh Robin
(86) International application number: PCT/US2005/039282
(87) International publication number: WO 2006/050270

(56) References cited:
- WO-A-98/48630
- WO-A-2005/003172
- WO-A-2006/003179
- IGARASHI TAKEHITO ET AL: "Enhanced cytotoxicity of allogeneic NK cells with killer immunoglobulin-like receptor ligand incompatibility against melanoma and renal cell carcinoma cells" BLOOD, vol. 104, no. 1, 1 July 2004 (2004-07-01), pages 170-177, XP002382433 ISSN: 0006-4971
- VAN RHEE FRITS ET AL: "KIR-LIGAND mismatched natural killer cells effectively kill primary myeloma and myeloma cell lines." BLOOD, vol. 104, no. 11, Part 1, November 2004 (2004-11), pages 672A-673A, XP002382434 & 46TH ANNUAL MEETING OF THE AMERICAN-SOCIETY-OF-HEMATOLOGY; SAN DIEGO, CA, USA; DECEMBER 04 -07, 2004 ISSN: 0006-4971
- LEUNG WING ET AL: "Determinants of antileukemia effects of allogeneic NK cells." JOURNAL OF IMMUNOLOGY, vol. 172, no. 1, 1 January 2004 (2004-01-01), pages 644-650, XP002382435 ISSN: 0022-1767
- KOH C Y ET AL: "NK inhibitory-receptor blockade for purging of leukemia: effects on hematopoietic reconstitution" BIOLOGY OF BLOOD AND MARROW TRANSPLANTATION, KLUGE CARDEN JENNINGS PUBLISHING, CHARLOTTESVILLE, VA, US, vol. 8, no. 1, 2002, pages 17-25, XP002308879 ISSN: 1083-8791
- RUGGERI LOREDANA ET AL: "Role of natural killer cell alloreactivity in HLA-mismatched hematopoietic stem cell transplantation" BLOOD, vol. 94, no. 1, 1 July 1999 (1999-07-01), pages 333-339, XP002382436 ISSN: 0006-4971
- RABINOWICH H ET AL: "INCREASED PROLIFERATION, LYTIC ACTIVITY, AND PURITY OF HUMAN NATURAL KILLER CELLS COCULTURED WITH MITOGEN-ACTIVATED FEEDER CELLS" CELLULAR IMMUNOLOGY, ACADEMIC PRESS, SAN DIEGO, CA, US, vol. 135, no. 2, 1991, pages 454-470, XP001120159 ISSN: 0008-8749
- RUGGERI LOREDANA ET AL: "Effectiveness of donor natural killer cell alloreactivity in mismatched hematopoietic transplants" SCIENCE (WASHINGTON D C), vol. 295, no. 5562, 15 March 2002 (2002-03-15), pages 2097-2100, XP002382437 ISSN: 0036-8075
- ZAMBELLO R ET AL: "Expression and function of KIR and natural cytotoxicity receptors in NK-type lymphoproliferative diseases of granular lymphocytes" BLOOD, W.B.SAUNDERS COMPANY, ORLANDO, FL, US, vol. 102, no. 5, 1 September 2003 (2003-09-01), pages 1797-1805, XP002340119 ISSN: 0006-4971
- GIEBEL SEBASTIAN ET AL: "Survival advantage with KIR ligand incompatibility in hematopoietic stem cell transplantation from unrelated donors." BLOOD, vol. 102, no. 3, 1 August 2003 (2003-08-01), pages 814-819, XP002382438 ISSN: 0006-4971
- MALMBERG K-J ET AL: "KIR-ligand mismatch in allogeneic hematopoietic stem cell transplantation" MOLECULAR IMMUNOLOGY, ELMSFORD, NY, US, vol. 42, no. 4, February 2005 (2005-02), pages 531-534, XP004686649 ISSN: 0161-5890
- FARAG S S ET AL: "Natural killer cell receptors: New biology and insights into the graft-versus-leukemia effect" BLOOD, W.B.SAUNDERS COMPANY, ORLANDO, FL, US, vol. 100, no. 6, 15 September 2002 (2002-09-15), pages 1935-1947, XP002308880 ISSN: 0006-4971
- MORETTA A ET AL: "Receptors for HLA class-I molecules in human natural killer cells" ANNUAL REVIEW OF IMMUNOLOGY, ANNUAL REVIEWS INC, US, vol. 14, 1996, pages 619-648, XP002308882 ISSN: 0732-0582
- PIEKARZ R ET AL: "A review of depsipeptide and other histone deacetylase inhibitors in clinical trials", CURRENT PHARMACEUTICAL DESIGN, BENTHAM SCIENCE PUBLISHERS, NL, vol. 10, no. 19, 1 January 2004 (2004-01-01), pages 2289-2298, XP008111360, ISSN: 1381-6128
- ADAMS J: "Proteasome inhibitors as therapeutic agents", EXPERT OPINION ON THERAPEUTIC PATENTS, INFORMA HEALTHCARE, GB, vol. 13, no. 1, 1 January 2003 (2003-01-01), pages 45-57, XP002310609, ISSN: 1354-3776, DOI: DOI:10.1517/13543776.13.1.45

## Description

### CROSS REFERENCE TO RELATED APPLICATION

This application is related to U.S. Provisional Application No. 60/624,803, filed November 2, 2004.

### FIELD

The invention generally relates to a composition comprising an enriched NK cell population. The invention further relates to compositions for use in a method of treating a solid tumor or a hyperproliferative disorder by administering the enriched NK cell population to a mammalian subject in need thereof.

### BACKGROUND

Natural killer (NK) cells have antigen independent tumor cytotoxicity and have been shown in murine models to control and prevent tumor growth and dissemination. Moretta, et al., Nat. Immunol., 3: 6-8, 2002; Kim, et al., Proc. Natl. Acad. Sci. U.S.A., 97: 2731-2736, 2000. However, the exact role that NK cells play in the control of cancer in humans remains controversial.

NK cells do not re-arrange genes coding for specific antigen receptors, rather their recognition of targets is regulated through a balance of activating or inhibitory signals. Moretta, et al., Annu. Rev. Immunol., 19: 197-223, 2001. Importantly, NK cell inactivation is required to prevent their destruction of normal host tissues. Vilches, et al., Annu. Rev. Immunol., 20: 217-251, 2002. Therefore, even in the presence of an activating ligand, inhibitory ligands expressed on cells may deliver overriding signals culminating in a net suppression ofNK cell function. Recently, a growing number of NK cell inhibitory and activating receptors have been characterized. Yokoyama, et al., Semin. Immunol., 7: 89-101, 1995; Takei, et al., Immunol. Rev., 155: 67-77, 1997; Lanier, et al., Annu. Rev. Immunol., 16: 359-393, 1998; Long, et al., Immunol. Rev., 181: 223-233, 2001. NK cells can recognize MHC class I and class I-like molecules, through killer immunoglobulin (Ig)-like receptors (KIRs) expressed on their surface. Ligation of KIR by MHC class I on both normal and malignant tissues suppresses NK cell function. MHC class I molecules fall into groups that serve as ligands for specific KIR, resulting in inhibition of NK cell mediated cytotoxicity. For HLA-C, polymorphisms in amino acids residing in positions 77 and 80 dictate specificity for its target KIR. Moretta, et al., J. Exp. Med., 182: 875-884, 1995; Winter, et al., J. Immunol., 158: 4026-4028, 1997; Winter, et al., J. Immunol., 161: 571-577, 1998. KIR2DL1 recognizes group 2 HLA-C molecules that have an asparagine at position 77 and lysine at position 80 while KIR2DL2 and KIR2DL3 recognize group 1 HLA-C molecules that have a serine at position 77 and an asparagine at position 80.

The inactivation ofNK cells by self-HLA molecules might be a mechanism permitting malignantly transformed host cells to evade NK cell-mediated immunity. Because tumor-KIR ligands are always matched to NK cell KIR, autologous NK cells would be inhibited by MHC class I expressing tumors, even in the presence of activating ligands. This may in part explain the failure of adoptively transfused autologous NK (Frohn, et al., J. Immunother., 23: 499-504, 2000) or lymphokine activated killer cells (LAK) to mediate anti-tumor effects against most metastatic solid tumors. Rosenberg, et al., N. Engl. J. Med., 313: 1485-1492, 1985. Only tumor cells which have lost expression of MHC class I or possess a dominant activating ligand would be predicted to be susceptible to such populations. Liao, et al., Science, 253: 199-202, 1991; Giebel, et al., Blood, 102: 814-819, 2003; Childs, et al., N. Engl. J. Med., 343: 750-758, 2000; Rini, et al., J. Urol., 165: 1208-1209, 2001; Bregni, et al., Blood, 99: 4234-4236, 2002; Ueno, et al., J. Clin. Oncol., 16: 986-993, 1998; Hentschke, et al., Bone Marrow Transplant, 31: 253-261, 2003; Strair, et al., J. Clin. Oncol., 21: 3785-3791, 2003.

A need exists in the art to find compositions and methods to prevent or override natural killer cell inactivation by self HLA molecules or MHC class I expressing tumors. Inactivation of NK cells potentially allows neoplastic cells to evade host NK-cell-mediated immunity.

### SUMMARY

The present invention is defined in the claims and generally relates to a composition comprising an enriched NK cell population. A method of treating a solid tumor or a hyperproliferative disorder which comprises administering the enriched NK cell population to a mammalian subject in need thereof is provided. The present invention provides a composition comprising an enriched allogeneic NK cell population for use as described in the claims. The enriched allogeneic NK cell population further comprises an enriched allogeneic KIR/KIR ligand-incompatible NK cell population. The present invention further provides a composition comprising an enriched autologous KIR/KIR ligand-incompatible NK cell population for use as described in the claims.

A method of treating a solid tumor is provided comprising administering to a mammalian subject a potentiating agent, e.g., a chemical agent or chemotherapeutic agent, and a composition comprising an enriched allogeneic NK cell population, in an amount effective to treat the solid tumor or to prevent the recurrence of the solid tumor. The potentiating agent acts to sensitize the tumor to killing by NK cells. In one aspect, the potentiating agent can be a chemotherapeutic agent or other tumor sensitizing agent. A method of treating a solid tumor is provided comprising administering to a mammalian subject a potentiating agent and a composition comprising an enriched autologous NK cell population, in an amount effective to treat a solid tumor or prevent the recurrence of the solid tumor.

A method of treating a solid tumor comprises administering to a mammalian subject compositions of the present invention. The present invention provides compositions for use in methods utilizing a composition comprising allogeneic NK-cells mismatched for KIR/KIR-ligands ("KIR/KIR ligand incompatible") which are more cytotoxic to solid tumor cells *in vitro* than their KIR/KIR-ligand matched autologous and allogeneic counterparts. The present invention further provides allogeneic NK populations, for use as described in the claims, enriched and cloned from the blood of cancer patients or healthy donors homozygous for HLA-C alleles in either group-1 (C-G1) or group-2 (C-G2) having increased cytotoxicity against EBV-LCL, renal cell carcinoma (RCC) and melanoma (MEL) cells lacking a matching KIR-inhibitory HLA-C ligand, while having only minimal cytotoxicity against KIR-ligand matched targets. This cytotoxic effect is preserved for at least 48 hours following NK cell gamma irradiation.

The present invention further provides a minority population of "autologous CD158a positive or CD158b positive KIR incompatible NK cells" (AKI-NK cells) for use as described in the claims that are "KIR/KIR ligand incompatible" and can be isolated and expanded *in vitro* from patients who are homozygous for HLA-C alleles in either group-1 (C-G1) or group-2 (C-G2); these AKI-NK cells, were shown to have enhanced cytotoxicity *in vitro* against autologous tumor cells and EBV-LCL compared to autologous "bulk" NK cells and other autologous NK cell subsets.

A method is provided to expand a high percentage of NK cells, *e.g.*, CD3⁻CD16⁺ NK cells, CD3⁻CD56⁺ NK cells, or KIR2DL2/3 positive NK cells. A significantly higher percentage of KIR 2DL2/3 positive NK cells can be expanded using EBV LCL feeder cells that either lack or have heterozygous expression for HLA-C alleles known to ligate KIR2DL2/3.

A method of treating a solid tumor is provided comprising administering to a mammalian subject a composition comprising an enriched allogeneic NK cell population, in an amount effective to reduce or eliminate the solid tumor or to prevent its occurrence or recurrence. The method further comprises gamma-irradiating the NK cell population prior to administering the composition to the mammalian subject. The method further comprises administering the gamma-irradiated NK cell population to the subject up to 48 hours following gamma-irradiation of the NK cells.

A method of treating a solid tumor is provided comprising administering to a mammalian subject a potentiating agent and a composition comprising an enriched allogeneic NK cell population, in an amount effective to reduce or eliminate the solid tumor or to prevent its occurrence or recurrence. The method further comprises gamma-irradiating the NK cell population prior to administering the composition to the mammalian subject. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor. In a further detailed aspect, the potentiating agent can be 5-fluorouracil.

A method of treating a solid tumor is provided comprising administering to a mammalian subject a potentiating agent and a composition comprising an enriched autologous NK cell population, in an amount effective to reduce or eliminate the solid tumor or to prevent its occurrence or recurrence. The method further comprises gamma-irradiating the NK cell population prior to administering the composition to the mammalian subject. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor. In a further detailed aspect, the potentiating agent can be 5-fluorouracil.

A method of treating a solid tumor is provided comprising administering to a mammalian subject a composition comprising an enriched autologous KIR/KIR ligand-incompatible NK cell population, in an amount effective to reduce or eliminate the solid tumor or to prevent its occurrence or recurrence. The method further comprises gamma-irradiating the NK cell population prior to administering the composition to the mammalian subject.

A method of treating or preventing a hyperproliferative disorder is provided comprising administering to a mammalian subject a composition comprising an enriched allogeneic NK cell population, in an amount effective to reduce or eliminate the solid tumor or to prevent its occurrence or recurrence. The method further comprises gamma-irradiating the NK cell population prior to administering the composition to the mammalian subject.

A method of treating or preventing a hyperproliferative disorder is provided comprising administering to a mammalian subject a potentiating agent and a composition comprising an enriched allogeneic NK cell population, in an amount effective to reduce or eliminate the solid tumor or to prevent its occurrence or recurrence. The method further comprises gamma-irradiating the NK cell population prior to administering the composition to the mammalian subject. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor.

A method of treating or preventing a hyperproliferative disorder is provided comprising administering to a mammalian subject a potentiating agent and a composition comprising an enriched autologous NK cell population, in an amount effective to reduce or eliminate the hyperproliferative disorder or to prevent its occurrence or recurrence. The method further comprises gamma-irradiating the NK cell population prior to administering the composition to the mammalian subject. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor.

A method of treating or preventing a hyperproliferative disorder is provided comprising administering to a mammalian subject a composition comprising an enriched autologous KIR/KIR ligand-incompatible NK cell population, in an amount effective to reduce or eliminate the hyperproliferative disorder or to prevent its occurrence or recurrence. The method further comprises gamma-irradiating the NK cell population prior to administering the composition to the mammalian subject. In one aspect, the hyperproliferative disorder is hematologic malignancy, solid tumors, melanoma, or genitourinary malignancy. In a detailed aspect, the genitourinary malignancy is bladder, urinary, kidney, testes, or prostate malignancy.

A in another embodiment, a method for inducing tumor cell death or inhibiting tumor cell proliferation in a mammalian subject comprises contacting or exposing the tumor cell to a composition comprising an enriched allogeneic NK cell population in an amount effective to induce the tumor cell death or inhibit the tumor cell proliferation in the mammalian subject. In one aspect, the tumor is a hematologic tumor, solid tumor, melanoma, or genitourinary malignancy. In a detailed aspect, the genitourinary malignancy is bladder cancer, urinary cancer, kidney cancer, testes cancer, or prostate cancer.

A In another embodiment, a method for inducing tumor cell death or inhibiting tumor cell proliferation in a mammalian subject comprises contacting or exposing the tumor cell to a potentiating agent and a composition comprising an enriched allogeneic NK cell population, in an amount effective to induce the tumor cell death or inhibit the tumor cell proliferation in the mammalian subj ect. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor. In a further aspect, the tumor cell is in a bladder of the mammalian subject. The method further comprises enhancing cytotoxicity of an NK cell population by inhibiting ligation of KIR and KIR ligand. In a detailed aspect, the method comprises treating the NK cell population with interfering RNA (RNAi) to the KIR cytoplasmic domain, antisense RNA to the KIR cytoplasmic domain, ribozymes to the KIR cytoplasmic domain, or antibodies to the KIR extracellular domain or to the KIR cytoplasmic domain.

A method for inducing tumor cell death or inhibiting tumor cell proliferation in a mammalian subject comprising contacting or exposing the tumor cell to a potentiating agent and a composition comprising an enriched autologous NK cell population, in an amount effective to induce the tumor cell death or inhibit the tumor cell proliferation in the mammalian subject. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor. In a further aspect, the tumor cell is in a bladder of the mammalian subject.

In another embodiment, a method for inducing tumor cell death or inhibiting tumor cell proliferation in a mammalian subject comprises contacting or exposing the tumor cell to a potentiating agent and a composition comprising an enriched autologous NK cell population, in an amount effective to induce the tumor cell death or inhibit the tumor cell proliferation in the mammalian subject. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor. In a further aspect, the tumor cell is in a bladder of the mammalian subject.

A method for inducing tumor cell death or inhibiting tumor cell proliferation in a mammalian subject is provided comprising contacting or exposing the tumor cell to a composition comprising an enriched autologous KIR/KIR ligand-incompatible NK cell population in an amount effective to induce the tumor cell death or inhibit the tumor cell proliferation in the mammalian subject. In a further aspect, the tumor cell is in a bladder of the mammalian subject. In one aspect, the enriched autologous NK cell population has homozygous HLA-Cw loci in KIR group 1. The method of claim 49, wherein the homozygous HLA-Cw loci is Cw1, Cw3, Cw7, Cw8, Cw12, Cw13, Cw14, Cw1507, or Cw16. In a further aspect, the enriched autologous NK cell population has homozygous HLA-Cw loci in KIR group 2. The method of claim 50, wherein the homozygous HLA-Cw loci is Cw2, Cw0307, Cw0315, Cw4, Cw5, Cw6, Cw0707, Cw0709, Cw1205, Cw12041, Cw12042, Cw15, Cw1602, Cw17, or Cw18

In a further aspect, the enriched autologous NK cell population has homozygous HLA-B loci in Bw4 or Bw6. The method of claim 51, wherein the homozygous HLA-B loci is Bw-5, Bw-13, Bw-1513, Bw-1516, Bw-1517, Bw-1523, Bw-1524, Bw-17, Bw-21, Bw-27, Bw-37, Bw-38, Bw-44, Bw-47, Bw-49, Bw-51, Bw-52, Bw-53, Bw-57, Bw-58, Bw-59, Bw-63, or Bw-77. The method of claim 51, wherein the homozygous HLA-B loci is Bw-7, Bw- 8, Bw- 12, Bw- 14, Bw- 18, Bw- 2708, Bw- 35, Bw- 39, Bw- 40, Bw- 4005, Bw- 41, Bw- 42, Bw- 45, Bw-46, Bw- 48, Bw- 50, Bw- 54, Bw- 55, Bw- 56, Bw- 60, Bw- 61, Bw- 62, Bw- 64, Bw- 65, Bw-67, Bw- 71, Bw- 72, Bw- 73, Bw- 75, Bw- 76, Bw- 78, or Bw- 81. In a further aspect, the subject has a homozygous KIR ligand and is unable to turn off the NK population expressing a KIR that cannot be ligated by a self HLA molecule. The subject can have a HLA-B loci, for example, in Bw4 or Bw6. The subject can have a HLA-Cw loci, for example, in KIR group 1 or KIR group 2. The enriched autologous NK cell population can be homozygous HLA-Cw loci, for example, in KIR group 1 or in KIR group 2. The enriched autologous NK cell population can be heterozygous HLA-Cw loci, for example, in KIR group 1 or in KIR group 2.

In a further aspect, the method comprises enhancing cytotoxicity of an NK cell population by inhibiting ligation of KIR and KIR ligand. In a detailed aspect, themethod comprises treating the NK cell population with interfering RNA (RNAi) to the KIR cytoplasmic domain, antisense RNA to the KIR cytoplasmic domain, ribozymes to the KIR cytoplasmic domain, or antibodies to the KIR extracellular domain or to the KIR cytoplasmic domain.

A method for enhancing tumor cell cytotoxicity of an NK cell population is provided comprising treating the tumor cell with a potentiating agent. In one aspect, the potentiating agent sensitizes the tumor to killing by NK cells. The potentiating agent can be, for example, a chemical agent or a chemotherapeutic agent. In a detailed aspect, the potentiating agent can be a proteasome inhibitor, a histone deacetylase inhibitor.

A method of expanding a subpopulation ofNK cells from a mammalian subject is provided comprising expanding the NK cells using an EBV lymphoblastic cell line. In one aspect, the subpopulation is a KIR/KIR ligand incompatible NK cell population. In a detailed aspect, the KIR/KIR ligand incompatible NK cell population is KIR 2DL2/3. In another aspect, the NK cells are an autologous NK cell population. In a detailed aspect, the subpopulation of autologous NK cells is expanded at least 10⁴-fold.

A method of expanding a subpopulation ofNK cells from a mammalian subject is provided comprising expanding the NK cells using peripheral blood mononuclear cells. In one aspect, the subpopulation is a KIR/KIR ligand incompatible NK cell population. The KIR/KIR ligand incompatible NK cell population can express, for example, KIR 2DL2/3, KIR 2DL1, or NKB1. In a further aspect, the NK cells are an autologous NK cell population the subpopulation of autologous NK cells is expanded at least 10⁴-fold. In a detailed aspect, the subpopulation of autologous NK cells is expanded at least 10⁵-fold.

A method for enhancing cytotoxicity of an NK cell population is provided comprising inhibiting ligation of KIR and KIR ligand. The method further comprises enhancing NK cell cytotoxicity against a tumor by treating the NK cell population with interfering RNA (RNAi) to the KIR cytoplasmic domain. The method further comprises enhancing NK cell cytotoxicity against a tumor by treating the NK cells with antisense RNA to the KIR cytoplasmic domain. The method further comprises enhancing NK cell cytotoxicity by treating the NK cells with antibodies to the KIR extracellular domain or to the KIR cytoplasmic domain. In one aspect, the antibodies are monoclonal antibodies or single chain Fv antibodies. In a further aspect, the NK cell population is an allogeneic NK cell population. In a further aspect, the NK cell population is an autologous NK cell population.

A method for treating a solid tumor or hyperproliferative disease in a mammal is provided comprising administering to the mammal a nucleic acid molecule that hybridizes under stringent conditions to a killer immunoglobulin (Ig)-like receptor (KIR) target gene and attenuating expression of the target gene. In one aspect, the method further comprises contacting the nucleic acid molecule with an enriched allogeneic or autologous NK cell population. In a further aspect, the nucleic acid molecule is an antisense oligonucleotide. In a further aspect, the nucleic acid molecule is a double stranded RNA molecule. In a detailed aspect, the double stranded RNA molecule is short interfering RNA (siRNA) or short hairpin RNA (shRNA). In a further aspect, the nucleic acid molecule hybridizes to an intracellular domain of the KIR target gene.

In another embodiment, a method of inhibiting expression of a gene encoding a killer immunoglobulin (Ig)-like receptor (KIR) protein comprises the steps of (i) providing a biological system in which expression of a gene encoding the KIR protein is to be inhibited; (ii) contacting the system with an antisense oligonucleotide that hybridizes to a transcript encoding the KIR protein; and (iii) inhibiting expression of the gene encoding the KIR protein. In one aspect, the biological system is an enriched allogeneic or autologous NK cell population. In a further aspect, the method comprises administering the antisense oligonucleotide to an enriched NK cell population in a mammalian subject and increasing NK cell cytotoxicity to a solid tumor or hyperproliferative disease state.

In another embodiment, a method of inhibiting expression of a gene encoding a KIR protein comprises the steps of (i) providing a biological system in which expression of a gene encoding a KIR protein is to be inhibited; and (ii) contacting the system with a double stranded RNA molecule that hybridizes to a transcript encoding the KIR protein; and (iii) inhibiting expression of the gene encoding the KIR protein. In one aspect, the biological system is an enriched allogeneic or autologous NK cell population. In a detailed aspect, the method further comprises administering the double stranded RNA molecule to an enriched NK cell population in a mammalian subject and increasing NK cell cytotoxicity to a solid tumor or hyperproliferative disease state. In a further aspect, the double stranded RNA molecule is short interfering RNA (siRNA) or short hairpin RNA (shRNA).

### BRIEF DESCRIPTION OF THE DRAWINGS

Figures 1A, 1B, 1C, 1D show the isolation of CD 158b (KIR 2DL2/3) and CD 158a (KIR 2DL1) positive NK populations.
Figures 2A, 2B, 2C, 2D show RCC and melanoma cells are more susceptible to the cytotoxic effects of KIR-incompatible NK cell lines than KIR-matched lines.
Figures 3A, 3B, 3C show allogeneic KIR incompatible NK cell lines isolated by limiting dilution cloning have enhanced cytotoxicity against KIR-mismatched tumor targets.
Figures 4A, 4B, 4C show gamma irradiated NK cell maintain cytotoxicity against tumor cells for at least 48 hours following irradiation.
Figures 5A, 5B, 5C, 5D, 5E show the identification and expansion of a subdominant NK cell fraction with enhanced cytotoxicity against autologous tumor cells.
Figure 6 shows lentiviral transduction with RNAi that will knock down the inhibitory KIR long cytoplasmic domain, and proposed enhancement ofNK cell antitumor effects.
Figure 7 shows pretreatment of tumor cells with Depsipeptide or Velcade makes the tumor cells more susceptible to NK cell mediated lysis.
Figure 8 shows enhanced NK cell susceptibility of murine renal cell carcinoma (RENCA) pre-sensitized with Velcade or Depsipeptide.
Figure 9 shows enhanced allogeneic NK cell susceptibility of human renal cell carcinoma pre-sensitized with Velcade or Depsipeptide.
Figure 10 shows enhanced autologous NK cell susceptibility of human renal cell carcinoma pre-sensitized with Velcade or Depsipeptide.
Figure 11 shows enhanced autologous NK cell susceptibility of human renal cell carcinoma pre-sensitized with Velcade or Depsipeptide.
Figure 12 shows a time course to optimize chemical agent incubation with human renal cell carcinoma cell line.
Figure 13 shows that enhanced NK cell susceptibility of human renal cell carcinoma pre-sensitized with Velcade or Depsipeptide may occur via a mechanism involving TRAIL.
Figure 14 shows that enhanced NK cell susceptibility of human renal cell carcinoma pre-sensitized with Velcade or Depsipeptide may occur via a mechanism involving Fas ligand.
Figure 15 compares viability (apoptosis), proliferation, MHC Class I expression, and MIC-A/B expression in human renal cell carcinoma cell lines.
Figure 16 shows a human clinical trial to sensitize tumors in humans to NK cell attack.

### DETAILED DESCRIPTION

The invention is generally related to a composition comprising an enriched NK cell population as defined in the claims. A composition comprising an enriched allogeneic NK cell population is provided. The enriched allogeneic NK cell population further comprises KIR/KIR ligand-incompatible NK cells. A composition comprising an enriched autologous NK cell population is provided. The invention further relates to a composition for use in a method of treating a solid tumor comprising administering to a mammalian subject said composition of an enriched allogeneic or autologous NK cell population, as above, in an amount effective to treat the solid tumor or to prevent the recurrence of the solid tumor. Cellular inactivation through killer Ig-like receptors (KIR) potentially allows neoplastic cells to evade host NK-cell-mediated immunity. The present invention provides a composition comprising allogeneic NK-cells as defined in the claims mismatched for KIR-ligands which are more cytotoxic to solid tumor cells *in vitro* than their KIR-ligand matched autologous and allogeneic counterparts. The present invention further provides allogeneic NK populations as defined in the claims enriched and cloned from the blood of cancer patients or healthy donors homozygous for HLA-C alleles in either group-1 (C-G1) or group-2 (C-G2) having increased cytotoxicity against EBV-LCL, renal cell carcinoma (RCC) and melanoma (MBL) cells lacking a matching KIR-inhibitory HLA-C ligand, while having only minimal cytotoxicity against KIR-ligand matched targets. This cytotoxic effect is preserved for at least 48 hours following NK cell gamma irradiation.

The genes encoding KIR and their HLA-class I ligands are located on different chromosomes. As a consequence, NK-cells expressing KIR that cannot be ligated by self HLA molecules can also exist in healthy individuals and cancer patients. The present invention further provides a minority population of autologous CD158a positive or CD158b positive KIR incompatible NK cells (AKI-NK) as defined in the claims that can be isolated and expanded *in vitro* from patients who are homozygous for HLA-C alleles in either group-1 (C-G1) or group-2 (C-G2); these AKI-NK, were shown to have enhanced cytotoxicity *in vitro* against autologous tumor cells and EBV-LCL compared to autologous "bulk" NK cells and other autologous NK cell subsets. In 3 cancer patients and 2 healthy donors homozygous for HLA-C group I alleles, small populations (typically <1%) of CD3-/CD158a+/CD158b- AKI-NK cells were isolated by flow sorting. AKI-NK cells were expanded *in vitro* and in all 5 cases had greater cytotoxicity against autologous tumor cells and/or autologous EBV-LCL lines compared to CD3-/CD158a-/CD158b+ KIR-matched and "bulk" unselected autologous NK cell populations.

Based on these data, the present invention further provides compositions for use in a method of treating solid tumors by developing immunotherapy regimens to take advantage of the anti-neoplastic potential of adoptively infused allogeneic or autologous KIR-incompatible NK-cells into patients with solid tumors at an early or advanced stage.

It has been determined that:
1) Allogeneic NK cells with killer immunoglobulin-like receptor (KIR) ligand incompatibility to KIR have enhanced cytotoxicity against solid tumor cells compared to KIR matched populations
2) Autologous "KIR/KIR ligand incompatible" natural killer cell populations can be isolated and expanded in humans that have enhanced cytotoxicity against autologous solid tumor cells and/or EBV-lymphoblastic cell lines (EBV-LCL) compared to unselected "bulk" NK cell populations
3) Gamma irradiated NK cells retain significant cytotoxicity against tumor targets up to 48 hours following irradiation. Furthermore, they preserve their ability kill KIR incompatible tumor targets for at least 24 hours following irradiation.
4) A method has been developed to expand a high percentage ofNK cells, *e.g.*, CD3-CD16⁺ NK cells, CD3⁻CD56⁺ NK cells, or KIR2DL2/3 positive NK cells. NK cell populations can be rapidly expanded when irradiated EBV-LCL feeder cells are added to enriched NK cell cultures containing IL-2. Furthermore, when EBV-LCL cells are used as feeder cells that lack MHC-class I ligands for specific NK cell KIR, NK cell populations expressing a desired KIR were selectively expanded. For example, when group II homozygous EBV-LCL feeder cells (lacking expression of HLA-C alleles known to ligate KIR2DL2/3) or EBV-LCL feeders with heterozygous expression for HLA-C alleles are used for NK cell expansion, a greater percentage of KIR 2DL2/3 positive NK cells are selectively expanded.

"Natural killer (NK) cells" are cells of the immune system that kill target cells in the absence of a specific antigenic stimulus, and without restriction according to MHC class. Target cells can be tumor cells or cells harboring viruses. NK cells are characterized by the presence of CD56 and the absence of CD3 surface markers. NK cells typically comprise approximately 10 to 15% of the mononuclear cell fraction in normal peripheral blood. Historically, NK cells were first identified by their ability to lyse certain tumor cells without prior immunization or activation. NK cells are thought to provide a "back up" protective mechanism against viruses and tumors that might escape the CTL response by down regulating MHC class I presentation. In addition to being involved in direct cytotoxic killing, NK cells also serve a critical role in cytokine production, which can be important to control cancer, infection and can also be involved in fetal implantation.

"Cytotoxic" and "cytolytic", when used to describe the activity of effector cells such as NK cells, are intended to be synonymous. In general, cytotoxic activity relates to killing of target cells by any of a variety of biological, biochemical, or biophysical mechanisms. Cytolysis refers more specifically to activity in which the effector lyses the plasma membrane of the target cell, thereby destroying its physical integrity. This results in the killing of the target cell. Without wishing to be bound by theory, it is believed that the cytotoxic effect of NK cells is due to cytolysis.

"Activation of NK cells" refers to activation per se of the cytotoxic or cytostatic action of NK cells on foreign or abnormal cells or elevation of the cytotoxic or cytostatic action of pre-active (*i.e.*, already active) NK cells on foreign or abnormal cells, as well as elevation of their other biological functions, such as stimulation of cytokine production.

"Target cells refers to the cells that are killed by the cytotoxic activity of the NK cells of the invention. These include in particular cells that are malignant or otherwise derived from a cancer, and cells that are infected by pathogenic viruses such as HIV, EBV, CMV, or herpes.

"An enriched NK cell population" refers to an NK cell population selected for a sub-population of cells having a desirable anti-tumor/ cytotoxic activity and are selectable by a cellular marker, *e.g.*, a cell surface marker or intracellular marker. An enriched NK cell population can be isolated by binding a fluorescent probe to the cellular marker and selecting for the enriched NK cell population by fluorescence activated cell sorting (FACS) column, or negative depletion using immunomagnetic beads, or other cell selection and separation technique known in the art.

"Allogeneic" or "allogenic" refers to tissues, cells or DNA taken from different individuals of the same species who are not monozygotic (identical) twins. Two or more individuals, or cells from those individuals, are allogeneic when genes at one or more loci are not identical in sequence in each individual.

"Autologous" or "autogeneic" or "autogenous" refers to tissues, cells or DNA taken from an individual's own tissues. For example, in an autologous transfer or transplantation of NK cells, the donor and recipient are the same person. "Autologous" is related to self, or originating within an organism itself.

"KIR/KIR ligand-incompatible NK cell population" refers to NK cells that retain NK cell cytotoxicity, because the NK cell population fails to recognize MHC class I and class I-like molecules, for example, on tumor cells or malignant tissue, through killer immunoglobulin (Ig)-like receptors (KIRs) expressed on the NK cell surface. Ligation of KIR by MHC class I on both normal and malignant tissues can suppresses NK cell function, except in the case of KIR/KIR ligand-incompatibility. MHC class I molecules fall into groups that serve as ligands for specific KIR, resulting in inhibition ofNK cell mediated cytotoxicity. For example, in MHC class I HLA-C, polymorphisms in amino acids residing in positions 77 and 80 dictate specificity for its target KIR.

"KIR ligand" refers to a molecule that binds to killer immunoglobulin (Ig)-like receptors (KIRs), for example, an MHC class I molecule on the surface of a cell.

"Patient", "subject" or "mammal" are used interchangeably and refer to mammals such as human patients and non-human primates, as well as experimental animals such as rabbits, rats, and mice, and other animals. Animals include all vertebrates, *e.g.*, mammals and non-mammals, such as sheep, dogs, cows, chickens, amphibians, and reptiles.

"Treating" or "treatment" includes the administration of an enriched NK cell compositions, compounds or agents of the present invention to prevent or delay the onset of the symptoms, complications, or biochemical indicia of a disease, alleviating the symptoms or arresting or inhibiting further development of the disease, condition, or disorder (*e.g.*, cancer, metastatic cancer, or metastatic solid tumors). Treatment can be prophylactic, *i.e.*, adjuvant (to prevent or delay the onset of the disease, or to prevent the manifestation of clinical or subclinical symptoms thereof) or therapeutic suppression or alleviation of symptoms after the manifestation of the disease.

"An amount effective to reduce or eliminate the solid tumor or to prevent its occurrence or recurrence" or "an amount effective to reduce or eliminate the hyperproliferative disorder or to prevent its occurrence or recurrence" refers to an amount of a therapeutic compound that improves a patient outcome or survival following treatment for the tumor disease state or hyperproliferative disorder as measured by patient test data, survival data, elevation or suppression of tumor marker levels, reduced susceptibility based upon genetic profile or exposure to environmental factors.

"Cancer", "malignancy", "solid tumor" or "hyperproliferative disorder" are used as synonymous terms and refer to any of a number of diseases that are characterized by uncontrolled, abnormal proliferation of cells, the ability of affected cells to spread locally or through the bloodstream and lymphatic system to other parts of the body (*i.e.*, metastasize) as well as any of a number of characteristic structural and/or molecular features. A "cancerous" or "malignant cell" or "solid tumor cell" is understood as a cell having specific structural properties, lacking differentiation and being capable of invasion and metastasis. "Cancer" refers to all types of cancer or neoplasm or malignant tumors found in mammals, including carcinomas and sarcomas. Examples are cancers of the breast, lung, non-small cell lung, stomach, brain, head and neck, medulloblastoma, bone, liver, colon, genitourinary, bladder, urinary, kidney, testes, uterus, ovary, cervix, prostate, melanoma, mesothelioma, sarcoma,. (see DeVita, et al., (eds.), 2001, Cancer Principles and Practice of Oncology, 6th. Ed., Lippincott Williams & Wilkins, Philadelphia, PA.

"Cancer-associated" refers to the relationship of a nucleic acid and its expression, or lack thereof, or a protein and its level or activity, or lack thereof, to the onset of malignancy in a subject cell. For example, cancer can be associated with expression of a particular gene that is not expressed, or is expressed at a lower level, in a normal healthy cell. Conversely, a cancer-associated gene can be one that is not expressed in a malignant cell (or in a cell undergoing transformation), or is expressed at a lower level in the malignant cell than it is expressed in a normal healthy cell.

In the context of the cancer, the term "transformation" refers to the change that a normal cell undergoes as it becomes malignant. In eukaryotes, the term "transformation" can be used to describe the conversion of normal cells to malignant cells in cell culture.

"Hyperproliferative disease" refers to any disease or disorder in which the cells proliferate more rapidly than normal tissue growth. Thus, a hyperproliferating cell is a cell that is proliferating more rapidly than normal cells.

"Proliferating cells" are those which are actively undergoing cell division and growing exponentially. "Loss of cell proliferation control" refers to the property of cells that have lost the cell cycle controls that normally ensure appropriate restriction of cell division. Cells that have lost such controls proliferate at a faster than normal rate, without stimulatory signals, and do not respond to inhibitory signals.

"Advanced cancer" means cancer that is no longer localized to the primary tumor site, or a cancer that is Stage III or IV according to the American Joint Committee on Cancer (AJCC).

"Well tolerated" refers to the absence of adverse changes in health status that occur as a result of the treatment and would affect treatment decisions.

"Metastatic" refers to tumor cells, *e.g.*, human solid tumor or genitourinary malignancy, that are able to establish secondary tumor lesions in the lungs, liver, bone or brain of immune deficient mice upon injection into the mammary fat pad and/or the circulation of the immune deficient mouse.

### CANCER TREATMENT

A "solid tumor" includes, but is not limited to, sarcoma, melanoma, carcinoma, or other solid tumor cancer.

"Sarcoma" refers to a tumor which is made up of a substance like the embryonic connective tissue and is generally composed of closely packed cells embedded in a fibrillar or homogeneous substance. Sarcomas include, but are not limited to, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

"Melanoma" refers to a tumor arising from the melanocytic system of the skin and other organs. Melanomas include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

"Carcinoma" refers to a malignant new growth made up of epithelial cells tending to infiltrate the surrounding tissues and give rise to metastases. Exemplary carcinomas include, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypemephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidemoid carcinoma, carcinoma mucosum, mucous carcinoma, carcinoma myxomatodes, naspharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, and carcinoma viflosum.

"Leukemia" refers to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease--acute or chronic; (2) the type of cell involved; myeloid (myelogenous), lymphoid (lymphogenous), or monocytic; and (3) the increase or non-increase in the number of abnormal cells in the blood-leukemic or aleukemic (subleukemic). Leukemia includes, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Additional cancers include, for example, Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, neuroblastoma, esophageal cancer, genitourinary tract cancer, malignant hypercalcemia, cervical cancer, endometrial cancer, adrenal cortical cancer, and prostate cancer.

### POTENTIATING AGENTS

A method of treating a solid tumor is provided comprising administering to a mammalian subject a potentiating agent, *e.g.*, a chemical agent or chemotherapeutic agent and a composition comprising an enriched allogeneic NK cell population, in an amount effective to treat the solid tumor or to prevent the recurrence of the solid tumor. A method of treating a solid tumor is provided comprising administering to a mammalian subject a potentiating agent, e.g., a chemical agent or chemotherapeutic agent and a composition comprising an enriched autologous NK cell population, in an amount effective to treat a solid tumor or prevent the recurrence of the solid tumor. A "potentiating agent" refers to a compound that acts to improve a therapeutic profile by sensitizing the tumor to killing by NK cells. In one aspect, the potentiating agent can be a chemotherapeutic agent or other tumor-sensitizing agent. In a detailed aspect the potentiating agent can be a proteasome inhbitor, *e.g.*, Velcade (bortezamid), or a histone deacetylase, *e.g.*, depsipeptide. In a further detailed aspect, the potentiating agent can be 5-fluorouracil.

A potentiating agent, e.g., a chemical agent or chemotherapeutic agent refers chemical compounds or drugs useful in the treatment of disease. Chemical agents or chemotherapeutic agents act to sensitize the tumor to killing by NK cells in the treatment of disease, for example, neoplastic disease or cancer. Cancer chemotherapeutic agents are also commonly referred to as antineoplastic agents. There are a number of classes of chemotherapeutic compounds, encompassing nearly 100 individual drugs, as well as numerous drug combination therapies, methods of delivery and schedules of treatment. Each of these chemotherapeutic agents may be classified according to several criteria, such as class of compound and disease state treated. Certain agents have been developed to take advantage of the rapid division of cancer cells and target specific phases in the cell cycle, providing another method of classification. Agents can also be grouped according to the type and severity of their side effects or method of delivery. However, the most common classification of chemotherapeutic agents is by class of compound, which broadly encompasses the mechanism of action of these compounds.

Depending on the reference source consulted, there are slight differences in the classification of antineoplastics. The classes of compounds include, but are not limited to: proteasome inhibitors, histone deacetylase inhibitors, alkaloids; alkylating agents; anti-tumor antibiotics; antimetabolites; hormones and hormone analogs; immunomodulators; photosensitizing agents; and miscellaneous other agents. See U.S. Patent No. 6,911,200.

Proteasome inhibitors include, but are not limited to, Velcade® (bortezamid); PSI (N-carbobenzoyl-Ile-Glu-(OtBu)-Ala-Leu-CHO); MG-132 (N-carbobenzoyl-Leu-Leu-Leu-CHO); MG-115 (N-carbobenzoyl-Leu-Leu-Nva-CHO); MG-101 or Calpain Inh I (N-Acetyl-Leu-Leu-norLeu-CHO); ALLM (N-Acetyl-Leu-Leu-Met-CHO; N-carbobenzoyl-Gly-Pro-Phe-Leu-CHO; N-carbobenzoyl-Gly-Pro-Ala-Phe-CHO; N-carbobenzoyl-Leu-Leu-Phe-CHO; N-carbobenzoyl-Leu-Ala-Leu-CHO); Gliotoxin; SN50 NLS of NF-κB MW 2781; Bay 11-7082; Capsaicin; PDTC; ALLN; MG-262; PPM-18; Cyclosporin A; Epoxomicin

Inhibitors of histone deacetylase (HDAC) include, but are not limited to, the following structural classes: 1) hydroxamic acids, 2) cyclic peptides, 3) benzamides, and 4) short-chain fatty acids. See U.S. Patent No. 6,905,669. Cyclic peptides used as (HDAC) inhibitors are mainly cyclic tetrapeptides. Examples of cyclic peptides include, but are not limited to, Depsipeptide, trapoxin A, apicidin and FR901228. Trapoxin A is a cyclic tetrapeptide that contains a 2-amino-8-oxo-9,10-epoxydecanoyl (AOE) moiety. Kijima et al., J. Biol. Chem. 268: 22429-22435, 1993. Apicidin is a fungal metabolite that exhibits potent, broad-spectrum antiprotozoal activitity and inhibits HDAC activity at nanomolar concentrations. Darkin-Rattray et al., Proc. Natl. Acad. Sci. USA 93: 13143-13147, 1996. FR901228 is a depsipeptide that is isolated from *Chromobacterium violaceum*, and has been shown to inhibit HDAC activity at micromolar concentrations.

### DETECTABLE LABEL

The particular label or detectable group used in the assay can be detectable by spectroscopic, photochemical, biochemical, immunochemical, electrical, optical or chemical means. The particular type of label is not a critical aspect of the invention, so long as it does not significantly interfere with the specific binding of an antibody to the cellular marker on the NK cell used in the assay. The detectable group can be any material having a detectable physical or chemical property. Such detectable labels have been well-developed in the field of assays or immunoassays and, in general, most any label useful in such methods can be applied to the present invention. Thus, a label is any composition detectable by spectroscopic, photochemical, biochemical, immunochemical, electrical, optical or chemical means. Useful labels in the present invention include magnetic beads (*e.g.* Dynabeads™), fluorescent dyes (*e.g.*, fluorescein isothiocyanate, Texas red, rhodamine, and the like), radiolabels (*e.g.*, ³H,¹⁴C, ³⁵S, ¹²⁵I, ¹²¹I, ¹¹²In, ⁹⁹mTc), other imaging agents such as microbubbles (for ultrasound imaging), ¹⁸F, ¹¹C, ¹⁵O, (for Positron emission tomography), ^{99m}TC, ¹¹¹In (for Single photon emission tomography), enzymes (*e.g.*, horse radish peroxidase, alkaline phosphatase and others commonly used in an ELISA), and calorimetric labels such as colloidal gold or colored glass or plastic (*e.g.* polystyrene, polypropylene, latex, and the like) beads. Patents that described the use of such labels include U.S. Pat. Nos. 3,817,837; 3,850,752; 3,939,350; 3,996,345; 4,277,437; 4,275,149; and 4,366,241. See also Handbook of Fluorescent Probes and Research Chemicals (6th Ed., Molecular Probes, Inc., Eugene OR).

The label can be coupled directly or indirectly to the desired component of the assay according to methods well known in the art. As indicated above, a wide variety of labels can be used, with the choice of label depending on sensitivity required, ease of conjugation with the compound, stability requirements, available instrumentation, and disposal provisions.

Non-radioactive labels are often attached by indirect means. Generally, a ligand molecule (*e.g.*, biotin) is covalently bound to the molecule. The ligand then binds to an anti-ligand (*e.g.*, streptavidin) molecule which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, any haptenic or antigenic compound can be used in combination with an antibody.

The molecules can also be conjugated directly to signal generating compounds, *e.g.*, by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, and the like Chemiluminescent compounds include luciferin, and 2,3-dihydrophthalazinediones, *e.g.*, luminol. For a review of various labeling or signal producing systems which can be used, see, U.S. Pat. No. 4,391,904.

Means of detecting labels are well known to those of skill in the art. Thus, for example, where the label is a radioactive label, means for detection include a scintillation counter or photographic film as in autoradiography. Where the label is a fluorescent label, it can be detected by exciting the fluorochrome with the appropriate wavelength of light and detecting the resulting fluorescence. The fluorescence can be detected visually, by means of photographic film, by the use of electronic detectors such as charge coupled devices (CCDs) or photomultipliers and the like. Similarly, enzymatic labels can be detected by providing the appropriate substrates for the enzyme and detecting the resulting reaction product. Finally simple calorimetric labels can be detected simply by observing the color associated with the label. Thus, in various dipstick assays, conjugated gold often appears pink, while various conjugated beads appear the color of the bead.

Some assay formats do not require the use of labeled components. For instance, agglutination assays can be used to detect the presence of the target antibodies. In this case, antigen-coated particles are agglutinated by samples comprising the target antibodies. In this format, none of the components need be labeled and the presence of the target antibody is detected by simple visual inspection.

Frequently, the cellular marker and antibodies to the cellular marker will be labeled by joining, either covalently or non-covalently, a substance which provides for a detectable signal.

### TREATMENT REGIMES

The disclosure provides pharmaceutical compositions comprising an enriched NK cell population for the treatment of disease, *e.g.*, metastic cancer, solid tumors, or a hyperproliferative disorder, formulated together with a pharmaceutically acceptable carrier. Some compositions include a combination of multiple (*e.g.*, two or more) enriched NK cell populations of the invention.

In prophylactic applications, pharmaceutical compositions or medicaments are administered to a patient susceptible to, or otherwise at risk of a disease or condition (*i.e.*, a hyperproliferative disease or solid tumor) in an amount sufficient to eliminate or reduce the risk of recurrence of the hyperproliferative disease or solid tumor, lessen the severity, or delay the outset of the disease, including biochemical, histologic and/or behavioral symptoms of the disease, its complications and intermediate pathological phenotypes presenting during development of the disease. In therapeutic applications, compositions or medicants are administered to a patient suspected of, or already suffering from such a disease in an amount sufficient to cure, or at least partially arrest, the symptoms of the disease (biochemical, histologic and/or behavioral), including its complications and intermediate pathological phenotypes in development of the disease. An amount adequate to accomplish therapeutic or prophylactic treatment is defined as a therapeutically- or prophylactically-effective dose. In both prophylactic and therapeutic regimes, agents are usually administered in several dosages until a sufficient anti-proliferative response has been achieved. Typically, the anti-proliferative response is monitored and repeated dosages are given if the anti-proliferative response starts to wane.

### RNA AND DNA INTERFERENCE METHODS

### Short Interfering RNAs (RNAi)

RNA interference (RNAi) is a mechanism of post-transcriptional gene silencing mediated by double-stranded RNA (dsRNA), which is distinct from antisense and ribozyme-based approaches (see Jain, K.K., 2004, Pharmacogenomics 5:239-42 for a review ofRNAi and siRNA). RNA interference is useful in a method for treating a tumor or hyperproliferative disease state in a mammal by administering to the mammal a nucleic acid molecule (*e.g.*, dsRNA) that hybridizes under stringent conditions to a killer immunoglobulin (Ig)-like receptor (KIR) target gene, and attenuates expression of the target gene. dsRNA molecules are believed to direct sequence-specific degradation of mRNA in cells of various types after first undergoing processing by an RNase III-like enzyme called DICER (Bernstein et al., 2001, Nature 409:363) into smaller dsRNA molecules comprised of two 21 nt strands, each of which has a 5' phosphate group and a 3' hydroxyl, and includes a 19 nt region precisely complementary with the other strand, so that there is a 19 nt duplex region flanked by 2 nt-3' overhangs. RNAi is thus mediated by short interfering RNAs (siRNA), which typically comprise a double-stranded region approximately 19 nucleotides in length with 1-2 nucleotide 3' overhangs on each strand, resulting in a total length of between approximately 21 and 23 nucleotides. In mammalian cells, dsRNA longer than approximately 30 nucleotides typically induces nonspecific mRNA degradation via the interferon response. However, the presence of siRNA in mammalian cells, rather than inducing the interferon response, results in sequence-specific gene silencing.

In general, a short, interfering RNA (siRNA) comprises an RNA duplex that is preferably approximately 19 basepairs long and optionally further comprises one or two single-stranded overhangs or loops. An siRNA may comprise two RNA strands hybridized together, or may alternatively comprise a single RNA strand that includes a self-hybridizing portion. siRNAs may include one or more free strand ends, which may include phosphate and/or hydroxyl groups. siRNAs typically include a portion that hybridizes under stringent conditions with a target transcript. One strand of the siRNA (or, the self-hybridizing portion of the siRNA) is typically precisely complementary with a region of the target transcript, meaning that the siRNA hybridizes to the target transcript without a single mismatch. In certain embodiments of the invention in which perfect complementarity is not achieved, it is generally preferred that any mismatches be located at or near the siRNA termini.

siRNAs have been shown to downregulate gene expression when transferred into mammalian cells by such methods as transfection, electroporation, or microinjection, or when expressed in cells via any of a variety of plasmid-based approaches. RNA interference using siRNA is reviewed in, *e.g.*, Tuschl, T., 2002, Nat. Biotechnol. 20: 446-448; See also Yu, J., et al., 2002, Proc. Natl. Acad. Sci., 99:6047-6052; Sui, G., et al., 2002, Proc. Natl. Acad. Sci USA., 99:5515-5520; Paddison, P., et al., 2002, Genes and Dev. 16, 948-958; Brummelkamp, T., et al., 2002, Science 296, 550-553; Miyagashi, M. and Taira, K., 2002, Nat. Biotech. 20:497-500; Paul, C., et al., 2002, Nat. Biotech. 20:505-508. As described in these and other references, the siRNA may consist of two individual nucleic acid strands or of a single strand with a self-complementary region capable of forming a hairpin (stem-loop) structure. A number of variations in structure, length, number of mismatches, size of loop, identity of nucleotides in overhangs, etc., are consistent with effective siRNA-triggered gene silencing. While not wishing to be bound by any theory, it is thought that intracellular processing (*e.g.*, by DICER) of a variety of different precursors results in production of siRNA capable of effectively mediating gene silencing. Generally it is preferred to target exons rather than introns, and it may also be preferable to select sequences complementary to regions within the 3' portion of the target transcript. Generally it is preferred to select sequences that contain approximately equimolar ratio of the different nucleotides and to avoid stretches in which a single residue is repeated multiple times.

siRNAs may thus comprise RNA molecules having a double-stranded region approximately 19 nucleotides in length with 1-2 nucleotide 3' overhangs on each strand, resulting in a total length of between approximately 21 and 23 nucleotides. As used herein, siRNAs also include various RNA structures that may be processed *in vivo* to generate such molecules. Such structures include RNA strands containing two complementary elements that hybridize to one another to form a stem, a loop, and optionally an overhang, preferably a 3' overhang. Preferably, the stem is approximately 19 bp long, the loop is about 1-20, more preferably about 4-10, and most preferably about 6-8 nt long and/or the overhang is about 1-20, and more preferably about 2-15 nt long. In certain embodiments of the disclosure the stem is minimally 19 nucleotides in length and may be up to approximately 29 nucleotides in length. Loops of 4 nucleotides or greater are less likely subject to steric constraints than are shorter loops and therefore may be preferred. The overhang may include a 5' phosphate and a 3' hydroxyl. The overhang may but need not comprise a plurality of U residues, *e.g.*, between 1 and 5 U residues. Classical siRNAs as described above trigger degradation of mRNAs to which they are targeted, thereby also reducing the rate of protein synthesis. In addition to siRNAs that act via the classical pathway, certain siRNAs that bind to the 3' UTR of a template transcript may inhibit expression of a protein encoded by the template transcript by a mechanism related to but distinct from classic RNA interference, *e.g.*, by reducing translation of the transcript rather than decreasing its stability. Such RNAs are referred to as microRNAs (mRNAs) and are typically between approximately 20 and 26 nucleotides in length, *e.g.*, 22 nt in length. It is believed that they are derived from larger precursors known as small temporal RNAs (stRNAs) or mRNA precursors, which are typically approximately 70 nt long with an approximately 4-15 nt loop. (See Grishok, A. et al., 2001, Cell 106:23-24; Hutvagner, G., et al., 2001, Science 293:834-838; Ketting, R., et al., Genes Dev., 15, 2654-2659). Endogenous RNAs of this type have been identified in a number of organisms including mammals, suggesting that this mechanism of post-transcriptional gene silencing may be widespread (Lagos-Quintana, M., et al., 2001, Science 294, 853-858; Pasquinelli, A., 2002, Trends in Genetics 18:171-173, and references in the foregoing two articles). MicroRNAs have been shown to block translation of target transcripts containing target sites in mammalian cells (Zeng, Y., et al., 2002, Molecular Cell 9: 1-20).

siRNAs such as naturally occurring or artificial (*i.e.*, designed by humans) mRNAs that bind within the 3' UTR (or elsewhere in a target transcript) and inhibit translation may tolerate a larger number of mismatches in the siRNA/template duplex, and particularly may tolerate mismatches within the central region of the duplex. In fact, there is evidence that some mismatches may be desirable or required as naturally occurring stRNAs frequently exhibit such mismatches as do mRNAs that have been shown to inhibit translation *in vitro.* For example, when hybridized with the target transcript such siRNAs frequently include two stretches of perfect complementarity separated by a region of mismatch. A variety of structures are possible. For example, the mRNA may include multiple areas of nonidentity (mismatch). The areas of nonidentity (mismatch) need not be symmetrical in the sense that both the target and the mRNA include nonpaired nucleotides. Typically the stretches of perfect complementarity are at least 5 nucleotides in length, *e.g.*, 6, 7, or more nucleotides in length, while the regions of mismatch may be, for example, 1, 2, 3, or 4 nucleotides in length.

Hairpin structures designed to mimic siRNAs and mRNA precursors are processed intracellularly into molecules capable of reducing or inhibiting expression of target transcripts (McManus, M. T., et al., 2002, RNA 8:842-850). These hairpin structures, which are based on classical siRNAs consisting of two RNA strands forming a 19 bp duplex structure are classified as class I or class II hairpins. Class I hairpins incorporate a loop at the 5' or 3' end of the antisense siRNA strand (*i.e.*, the strand complementary to the target transcript whose inhibition is desired) but are otherwise identical to classical siRNAs. Class II hairpins resemble mRNA precursors in that they include a 19 nt duplex region and a loop at either the 3' or 5' end of the antisense strand of the duplex in addition to one or more nucleotide mismatches in the stem. These molecules are processed intracellularly into small RNA duplex structures capable of mediating silencing. They appear to exert their effects through degradation of the target mRNA rather than through translational repression as is thought to be the case for naturally occurring mRNAs and stRNAs.

Thus it is evident that a diverse set of RNA molecules containing duplex structures is able to mediate silencing through various mechanisms. For the purposes of the present invention, any such RNA, one portion of which binds to a target transcript and reduces its expression, whether by triggering degradation, by inhibiting translation, or by other means, is considered to be an siRNA, and any structure that generates such an siRNA (*i.e.*, serves as a precursor to the RNA) is useful in the practice of the present invention.

In the context of the present invention, siRNAs are useful both for therapeutic purposes, *e.g.*, to modulate the expression of a KIR protein in a subject at risk of or suffering from a tumor or hyperproliferative disease state. In one embodiment, the therapeutic treatment of a solid tumor or hyperproliferative disorder with an enriched NK cell population in combination with an antibody, antisense vector, or double stranded RNA vector.

The invention therefore provides a method of inhibiting expression of a gene encoding an inhibitory KIR protein comprising the step of (i) providing a biological system in which expression of a gene encoding inhibitory KIR protein is to be inhibited; and (ii) contacting the system with an siRNA targeted to a transcript encoding the inhibitory KIR protein. According to certain embodiments of the invention the biological system comprises an NK cell, and the contacting step comprises expressing the siRNA in the cell. According to certain embodiments of the invention the biological system comprises a subject, *e.g.*, a mammalian subject such as a mouse or human, and the contacting step comprises administering the siRNA to the subject or comprises expressing the siRNA in the subject. According to certain embodiments of the invention the siRNA is expressed inducibly and/or in a cell-type or tissue specific manner.

By "biological system" is meant any vessel, well, or container in which biomolecules (*e.g.*, nucleic acids, polypeptides, polysaccharides, lipids, and the like) are placed; a cell or population of cells; a tissue; an organ; an organism, and the like. Typically the biological system is a cell or population of cells, *e.g.*, NK cells, but the method can also be performed in a vessel using purified or recombinant proteins.

The disclosure provides siRNA molecules targeted to a transcript encoding a KIR protein. In particular, the disclosure provides siRNA molecules selectively or specifically targeted to a transcript encoding a polymorphic variant of such a transcript, in an enriched NK cell population. The terms "selectively" or "specifically targeted to", in this context, are intended to indicate that the siRNA causes greater reduction in expression of the variant than of other variants (*i.e.*, variants whose existence in a subject is not indicative of susceptibility to or presence of a chemotherapy-resistant neoplastic disease). The siRNA, or collections of siRNAs, may be provided in the form of kits with additional components as appropriate.

### Short hairpin RNAs (shRNA)

RNA interference (RNAi),a mechanism of post-transcriptional gene silencing mediated by double-stranded RNA (dsRNA), is useful in a method for treating a neoplastic disease state in a mammal by administering to the mammal a nucleic acid molecule (*e.g.*, dsRNA) that hybridizes under stringent conditions to a killer immunoglobulin (Ig)-like receptor (KIR) target gene, and attenuates expression of said target gene. See Jain, K.K., 2004, Pharmacogenomics 5:239-42 for a review of RNAi and siRNA. A further method of RNA interference in the present invention is the use of short hairpin RNAs (shRNA). A plasmid containing a DNA sequence encoding for a particular desired siRNA sequence is delivered into a target cell via transfection or virally-mediated infection. Once in the cell, the DNA sequence is continuously transcribed into RNA molecules that loop back on themselves and form hairpin structures through intramolecular base pairing. These hairpin structures, once processed by the cell, are equivalent to transfected siRNA molecules and are used by the cell to mediate RNAi of the desired protein. The use of shRNA has an advantage over siRNA transfection as the former can lead to stable, long-term inhibition of protein expression. Inhibition of protein expression by transfected siRNAs is a transient phenomenon that does not occur for times periods longer than several days. In some cases, this may be preferable and desired. In cases where longer periods of protein inhibition are necessary, shRNA mediated inhibition is preferable.

### Full and Partial Length Antisense RNA Transcripts

Antisense RNA transcripts have a base sequence complementary to part or all of any other RNA transcript in the same cell. Such transcripts have been shown to modulate gene expression through a variety of mechanisms including the modulation of RNA splicing, the modulation of RNA transport and the modulation of the translation of mRNA (Denhardt, 1992, Ann N Y Acad. Sci. 660:70; Nellen, 1993, Trends Biochem. Sci. 18:419; Baker and Monia, 1999, Biochim. Biophys. Acta, 1489:3; Xu, et al., 2000, Gene Therapy 7:438; French and Gerdes, 2000, Curr. Opin. Microbiol. 3:159; Terryn and Rouze, 2000, Trends Plant Sci. 5: 1360)

### Antisense RNA and DNA Oligonucleotides

Antisense nucleic acids are generally single-stranded nucleic acids (DNA, RNA, modified DNA, or modified RNA) complementary to a portion of a target nucleic acid (*e.g.*, an mRNA transcript) and therefore able to bind to the target to form a duplex. Typically they are oligonucleotides that range from 15 to 35 nucleotides in length but may range from 10 up to approximately 50 nucleotides in length. Binding typically reduces or inhibits the function of the target nucleic acid. For example, antisense oligonucleotides may block transcription when bound to genomic DNA, inhibit translation when bound to mRNA, and/or lead to degradation of the nucleic acid. Reduction in expression of an inhibitory KIR polypeptide may be achieved by the administration of antisense nucleic acids or peptide nucleic acids comprising sequences complementary to those of the mRNA that encodes the polypeptide. Antisense technology and its applications are well known in the art and are described in Phillips, M. I. (ed.) Antisense Technology, Methods Enzymol., 2000, Volumes 313 and 314, Academic Press, San Diego, and references mentioned therein. See also Crooke, S. (ed.), "Antisense Drug Technology: Principles, Strategies, and Applications" (1st Edition), Marcel Dekker; and references cited therein.

Antisense oligonucleotides can be synthesized with a base sequence that is complementary to a portion of any RNA transcript in the cell. Antisense oligonucleotides may modulate gene expression through a variety of mechanisms including the modulation of RNA splicing, the modulation of RNA transport and the modulation of the translation of mRNA (Denhardt, 1992). Various properties of antisense oligonucleotides including stability, toxicity, tissue distribution, and cellular uptake and binding affinity may be altered through chemical modifications including (i) replacement of the phosphodiester backbone (*e.g.*, peptide nucleic acid, phosphorothioate oligonucleotides, and phosphoramidate oligonucleotides), (ii) modification of the sugar base (*e.g.*, 2'-O-propylribose and 2'-methoxyethoxyribose), and (iii) modification of the nucleoside (*e.g.*, C-5 propynyl U, C-5 thiazole U, and phenoxazine C) (Wagner, 1995, Nat. Medicine 1:11116; Varga, et al., 1999, Immun. Lett. 69:217; Neilsen, Curr. Opin. Biotech. 10:71, 1999; Woolf, 1990, Nucleic Acids Res. 18:1763).

### Ribozymes

Certain nucleic acid molecules referred to as ribozymes or deoxyribozymes have been shown to catalyze the sequence-specific cleavage of RNA molecules. The cleavage site is determined by complementary pairing of nucleotides in the RNA or DNA enzyme with nucleotides in the target RNA. Thus, RNA and DNA enzymes can be designed to cleave to any RNA molecule, thereby increasing its rate of degradation (Cotten and Birnstiel, 1989, EMBO J. 8:3861-3866; Usman et al., 1996, Nucl. Acids Mol. Biol. 10:243; Usman, et al., 1996, Curr. Opin. Struct. Biol. 1:527; Sun, et al., 2000, Pharmacol. Rev., 52:325. See also *e.g.*, Cotten and Birnstiel, 1989, EMBO J. 8:3861-3866.)

### SCFV PHAGE LIBRARIES

A library of scFv antibodies to killer immunoglobulin (Ig)-like receptor (KIR) intracellular domain or extracellular domain can be used to treat a solid tumor or hyperproliferative disease. One approach for a phage display library to identity an antibody composition that specifically binds to a KIR protein, has been the use of scFv phage-libraries (see, *e.g.*, Huston et al., Proc. Natl. Acad. Sci U.S.A., 85: 5879-5883, 1988; Chaudhary et al., Proc. Natl. Acad. Sci U.S.A., 87: 1066-1070, 1990. Various embodiments of scFv libraries displayed on bacteriophage coat proteins have been described. Refinements of phage display approaches are also known, for example as described in WO96/06213 and WO92/01047 (Medical Research Council *et al.*) and WO97/08320 (Morphosys). The display of Fab libraries is also known, for instance as described in WO92/01047 (CAT/MRC) and WO91/17271 (Affymax).

Hybrid antibodies or hybrid antibody fragments that are cloned into a display vector can be selected against the appropriate antigen associated with KIR on an NK cell for treatment of a solid tumor or hyperproliferative disease in order to identify variants that maintained good binding activity because the antibody or antibody fragment will be present on the surface of the phage or phagemid particle. See for example Barbas III, et al., Phage Display, A Laboratory Manual, Cold Spring Harbor Laboratory Press, Cold Spring Harbor, N.Y., 2001. For example, in the case of Fab fragments, the light chain and heavy chain Fd products are under the control of a lac promoter, and each chain has a leader signal fused to it in order to be directed to the periplasmic space of the bacterial host. It is in this space that the antibody fragments will be able to properly assemble. The heavy chain fragments are expressed as a fusion with a phage coat protein domain which allows the assembled antibody fragment to be incorporated into the coat of a newly made phage or phagemid particle. Generation of new phagemid particles requires the addition of helper phage which contain all the necessary phage genes. Once a library of antibody fragments is presented on the phage or phagemid surface, a process termed panning follows. This is a method whereby i) the antibodies displayed on the surface of phage or phagemid particles are bound to the desired antigen, ii) non-binders are washed away, iii) bound particles are eluted from the antigen, and iv) eluted particles are exposed to fresh bacterial hosts in order to amplify the enriched pool for an additional round of selection. Typically three or four rounds of panning are performed prior to screening antibody clones for specific binding. In this way phage/phagemid particles allow the linkage of binding phenotype (antibody) with the genotype (DNA) making the use of antibody display technology very successful. However, other vector formats could be used for this humanization process, such as cloning the antibody fragment library into a lytic phage vector (modified T7 or Lambda Zap systems) for selection and/or screening.

After selection of desired hybrid antibodies and/or hybrid antibody fragments, it is contemplated that they can be produced in large volume by any technique known to those skilled in the art, *e.g.*, prokaryotic or eukaryotic cell expression and the like. For example, hybrid antibodies or fragments may be produced by using conventional techniques to construct an expression vector that encodes an antibody heavy chain in which the CDRs and, if necessary, a minimal portion of the variable region framework, that are required to retain original species antibody binding specificity (as engineered according to the techniques described herein) are derived from the originating species antibody and the remainder of the antibody is derived from a target species immunoglobulin which may be manipulated as described herein, thereby producing a vector for the expression of a hybrid antibody heavy chain.

In a detailed embodiment, a single-chain Fv (scFv) antibody library can be prepared from the peripheral blood lymphocytes of 5, 10, 15, or 20 or more patients with various cancer diseases. Completely human high-affinity scFv antibodies can then be selected by using synthetic sialyl Lewis^{x} and Lewis^{x} BSA conjugates. In one study, these human scFv antibodies were specific for sialyl Lewis^{x} and Lewis^{x}, as demonstrated by ELISA, BIAcore, and flow cytometry binding to the cell surface of pancreatic adenocarcinoma cells. Nucleotide sequencing revealed that at least four unique scFv genes were obtained. The K_{d} values ranged from 1.1 to 6.2 x 10⁻⁷ M that were comparable to the affinities of mAbs derived from the secondary immune response. These antibodies could be valuable reagents for probing the structure and function of carbohydrate antigens and in the treatment of human tumor diseases. Mao, et al., Proc. Natl. Acad. Sci. U.S.A. 96: 6953-6958, 1999.

In a further detailed embodiment, phage displayed combinatorial antibody libraries can be used to generate and select a wide variety of antibodies to an appropriate antigen associated with a metastatic cell, *e.g.*, a KIR protein on an NK cell. The phage coat proteins pVII and pIX can be used to display the heterodimeric structure of the antibody Fv region. Aspects of this technology have been extended to construct a large, human single-chain Fv (scFv) library of 4.5 x 10⁹ members displayed on pIX of filamentous bacteriophage. Furthermore, the diversity, quality, and utility of the library were demonstrated by the selection of scFv clones against six different protein antigens. Notably, more than 90% of the selected clones showed positive binding for their respective antigens after as few as three rounds of panning. Analyzed scFvs were also found to be of high affinity. For example, kinetic analysis (BIAcore) revealed that scFvs against staphylococcal enterotoxin B and cholera toxin B subunit had a nanomolar and subnanomolar dissociation constant, respectively, affording affinities comparable to, or exceeding that, of mAbs obtained from immunization. High specificity was also attained, not only between very distinct proteins, but also in the case of more closely related proteins, *e.g.*, *Ricinus communis* ("ricin") agglutinins (RCA₆₀ and RCA₁₂₀), despite >80% sequence homology between the two. The results suggested that the performance of pIX-display libraries can potentially exceed that of the pIII-display format and make it ideally suited for panning a wide variety of target antigens. Gao et al., Proc. Natl. Acad. Sci. U.S.A. 99: 12612-12616,2001.

Specific binding between an antibody or other binding agent and an antigen means a binding affinity of at least 10⁻⁶ M. Preferred binding agents bind with affinities of at least about 10⁻⁷ M, and preferably 10⁻⁸ M to 10⁻⁹ M, 10⁻¹⁰ M, 10⁻¹¹ M, or 10⁻¹² M. The term epitope means an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

### EFFECTIVE DOSAGES

Effective doses of a composition of an enriched NK cell population for the treatment of disease, *e.g.*, metastic cancer, solid tumors, or a hyperproliferative disorder, described herein vary depending upon many different factors, including means of administration, target site, physiological state of the patient, whether the patient is human or an animal, other medications administered, and whether treatment is prophylactic or therapeutic. Usually, the patient is a human but nonhuman mammals including transgenic mammals can also be treated. Treatment dosages need to be titrated to optimize safety and efficacy.

For administration with an enriched NK cell populations, the dosage ranges from about 1 x 10⁶ to about 1 x 10¹² NK cells per patient. For administration with an enriched NK cell populations, the dosage ranges from about 1 x 10⁵ to about 1 x 10⁹ NK cells per kilogram recipient weight, or the dosage ranges from about 5 x 10⁶ to about 1 x 10⁸ NK cells per kilogram recipient weight. An exemplary treatment regime entails administration once per every two weeks or once a month or once every 3 to 6 months. In some methods, two or more enriched NK cell populations are administered simultaneously, in which case the dosage of each enriched NK cell populations administered falls within the ranges indicated. Multiple administrations of enriched NK cell populations can occur. Intervals between single dosages can be weekly, monthly or yearly. Intervals can also be irregular as indicated by measuring blood levels of the enriched NK cell population in the patient. Alternatively, enriched NK cell populations can be administered as a sustained release formulation, in which case less frequent administration is required. Dosage and frequency vary depending on the half-life of the enriched NK cell populations in the patient. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

### ROUTES OF ADMINISTRATION

Compositions of an enriched NK cell population for the treatment of disease, *e.g.*, metastic cancer, solid tumors, or a hyperproliferative disorder, can be administered by intravenous, intravesicular, intrathecal, parenteral, topical, subcutaneous, oral, intranasal, intraarterial, intracranial, intraperitoneal, or intramuscular means. As a prophylactic/ adjuvant or for treatment of disease, enriched NK cell populations target a hyperproliferative disorder or solid tumor, *e.g.*, a genitourinary malignancy, and/or therapeutic treatment. The most typical route of administration of an immunogenic agent is subcutaneous although other routes can be equally effective. The next most common route is intramuscular injection. This type of injection is most typically performed in the arm or leg muscles. In some methods, agents are injected directly into a particular tissue where deposits have accumulated, for example intracranial injection. Intramuscular injection on intravenous infusion are preferred for administration of an enriched NK cell population. In some methods, a particular therapeutic enriched NK cell population is injected directly into the bladder.

Agents of the invention can optionally be administered in combination with other agents that are at least partly effective in treating various diseases including various hyperproliferative diseases. In the case of tumor metastasis to the brain, agents of the invention can also be administered in conjunction with other agents that increase passage of the agents of the invention across the blood-brain barrier (BBB).

### FORMULATION

Compositions of an enriched NK cell population for the treatment of disease, *e.g.*, metastic cancer, solid tumors, or a hyperproliferative disorder,

Compositions of an enriched NK cell population for the treatment of disease, *e.g.,* metastic cancer, solid tumors, or a hyperproliferative disorder, are often administered as pharmaceutical compositions comprising an active therapeutic agent, *i.e.,* and a variety of other pharmaceutically acceptable components. See, *e.g.,* Alfonso R Gennaro (ed), Remington: The Science and Practice of Pharmacy, (Formerly Remington's Pharmaceutical Sciences) 20th ed., Lippincott, Williams & Wilkins, 2003. The preferred form depends on the intended mode of administration and therapeutic application. The compositions can also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the combination. An example of such diluent is X-vivo 20 media (Cambrex Bio Science, Walkersville, MD) containing 10% heat inactivated human AB serum or 10% autologous serum. Further examples of such diluents are distilled water, physiological phosphate-buffered saline, Ringer's solutions, dextrose solution, and Hank's solution. In addition, the pharmaceutical composition or formulation can also include other carriers, adjuvants, or nontoxic, nontherapeutic, nonimmunogenic stabilizers and the like.

Pharmaceutical compositions can also include large, slowly metabolized macromolecules such as proteins, polysaccharides such as chitosan, polylactic acids, polyglycolic acids and copolymers (such as latex functionalized Sepharose™, agarose, cellulose, and the like), polymeric amino acids, amino acid copolymers, and lipid aggregates (such as oil droplets or liposomes). Additionally, these carriers can function as immunostimulating agents (*i.e.*, adjuvants).

For parenteral administration, compositions of the invention can be administered as injectable dosages of a solution or suspension of the substance in a physiologically acceptable diluent with a pharmaceutical carrier that can be a sterile liquid such as water oils, saline, glycerol, or ethanol. Additionally, auxiliary substances, such as wetting or emulsifying agents, surfactants, pH buffering substances and the like can be present in compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions. Enriched NK cell populations can be administered in the form of a depot injection or implant preparation which can be formulated in such a manner as to permit a sustained release of the active ingredient. An exemplary composition comprises an enriched NK cell population at 5 mg/mL, formulated in aqueous buffer consisting of 50 mM L-histidine, 150 mM NaCl, adjusted to pH 6.0 with HCl.

Typically, compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above. Langer, Science, 249: 1527, 1990; Hanes, Advanced Drug Delivery Reviews, 28: 97-119, 1997. The agents of this invention can be administered in the form of a depot injection or implant preparation which can be formulated in such a manner as to permit a sustained or pulsatile release of the active ingredient.

Additional formulations suitable for other modes of administration include oral, intranasal, and pulmonary formulations, suppositories, and transdermal applications.

For suppositories, binders and carriers include, for example, polyalkylene glycols or triglycerides; such suppositories can be formed from mixtures containing the active ingredient in the range of 0.5% to 10%, preferably 1%-2%. Oral formulations include excipients, such as pharmaceutical grades of mannitol, lactose, starch, magnesium stearate, sodium saccharine, cellulose, and magnesium carbonate. These compositions take the form of solutions, suspensions, tablets, pills, capsules, sustained release formulations or powders and contain 10%-95% of active ingredient, preferably 25%-70%.

Topical application can result in transdermal or intradermal delivery. Topical administration can be facilitated by co-administration of the agent with cholera toxin or detoxified derivatives or subunits thereof or other similar bacterial toxins. Glenn, et al., Nature, 391: 851, 1998. Co-administration can be achieved by using the components as a mixture or as linked molecules obtained by chemical crosslinking or expression as a fusion protein.

Alternatively, transdermal delivery can be achieved using a skin patch or using transferosomes. Paul, et al., Eur. J. Immunol., 25: 3521-24, 1995; Cevc, et al., Biochem. Biophys. Acta., 1368: 201-15, 1998.

The pharmaceutical compositions generally comprise a composition of the enriched NK cell population in a form suitable for administration to a patient. The pharmaceutical compositions are generally formulated as sterile, substantially isotonic and in full compliance with all Good Manufacturing Practice (GMP) regulations of the U.S. Food and Drug Administration.

### TOXICITY

Preferably, a therapeutically effective dose of a composition of the enriched NK cell population described herein will provide therapeutic benefit without causing substantial toxicity.

Toxicity of the enriched NK cell population described herein can be determined by standard pharmaceutical procedures in cell cultures or experimental animals, *e.g.,* by determining the LD₅₀ (the dose lethal to 50% of the population) or the LD₁₀₀ (the dose lethal to 100% of the population). The dose ratio between toxic and therapeutic effect is the therapeutic index. The data obtained from these cell culture assays and animal studies can be used in formulating a dosage range that is not toxic for use in human. The dosage of the enriched NK cell population described herein lies preferably within a range of circulating concentrations that include the effective dose with little or no toxicity. The dosage can vary within this range depending upon the dosage form employed and the route of administration utilized. The exact formulation, route of administration and dosage can be chosen by the individual physician in view of the patient's condition. (See, *e.g.,* Fingl, et al., The Pharmacological Basis Of Therapeutics, Ch. 1, 1975).

### KITS

Also within the scope of the invention are kits comprising the compositions (*e.g.*, an enriched NK cell population) of the invention and instructions for use. The kit can further contain a least one additional reagent, or one or more additional human antibodies of the invention (*e.g.*, a human antibody having a complementary activity which binds to an epitope in the antigen distinct from the first human antibody). Kits typically include a label indicating the intended use of the contents of the kit. The term label includes any writing, or recorded material supplied on or with the kit, or which otherwise accompanies the kit.

### EXEMPLARY EMBODIMENTS

### EXAMPLE 1

### Isolation and expansion of allogeneic KIR incompatible NK cells

*Generation of EBV transformed B-cell lines:* Epstein-Barr virus transformed lymphoblastic cell lines (EBV-LCL) were established by culturing peripheral blood mononuclear cells (PBMCs) in the presence of 100 µg/ml of Cyclosporine A (CSA, Sandoz Pharmaceuticals Inc, Washington, DC) with EBV supernatant harvested from the cell line B95-8 (ATCC, Manassas, Virginia). The human melanoma (MEL) cell lines St-MEL and R-MEL and the human renal cell carcinoma (RCC) cell line Wh-RCC were established from biopsies of metastatic lesions and a renal tumor primary specimen (National Cancer Institute [NCI]). The human RCC cell line SJ-RCC was established from a nephrectomy sample procured at the NCI and maintained in our laboratory. EBV-LCL, MEL, and RCC lines were maintained in RPMI 1640 with 10% fetal calf serum (FCS). MHC class I typing was performed at the National Institutes of Health HLA laboratory using polymerase chain reaction with sequence-specific primers (PCR-SSPs). Briefly, genomic DNA was first extracted from peripheral blood lymphocytes or tumor samples using primers and PCR conditions described by Bunce, et al., Tissue Antigens, 46: 355-367, 1995, incorporated herein by reference in its entirety. 23 The final PCR product was visualized on a 2% agarose gel stained with ethidium bromide, with results determined by the presence or absence of an appropriately sized band. For the purpose of this study, tumor lines were specifically selected to be homozygous for HLA-C alleles that would be predicted to ligate a single HLA-C-reactive KIR (either KIR2DL2/3 or KIR2DL1; Table 1).

**Table 1. HLA data on NK and tumor cells used in experiments**

| | NK Cells | Tumor cells | | | HLA class 1 | | | HLA-C group Homozygous |
|---|---|---|---|---|---|---|---|---|
| | | RCC | MEL | LCL | A | B | Cw | |
| R | - | - | + | + | 02,23 | 15, 35 | 02, 04 | 2 |
| ST | + | - | + | + | 02,29 | 07,08 | 0701, 0702 | 1 |
| Wh | - | + | - | - | 03, 01 | 08, 44 | 05, (-)+ | 2 |
| SJ | - | + | - | + | 11, 11 | 51, 55 | 01, 0303 | 1 |
| Volle | - | - | - | + | 02,03 | 07, 51 | 01, 0702 | 1 |
| KR0350 | + | - | - | + | 02,68 | 40, 44 | 0304, 0704 | 1 |
| SKEM* | + | - | - | - | 11,11 | 51, 55 | 01, 0303 | 1 |
| MORRC | + | - | - | - | 02, 11 | 35, 40 | 02, 04 | 2 |

| | | | | | | | | |
|---|---|---|---|---|---|---|---|---|
| HLA-C group patients are grouped according to HLA-C alleles are corresponding KIR ligands. *SKEM is an HLA-identical sibling donor for RCC patient SJ. +Wh-RCC lost an HLA-Cw locus. | | | | | | | | |

*NK cell culture media:* Bulk NK cells and NK cells isolated after limiting-dilution cloning were grown in Cellgro SCGM serum-free media (CellGenix, Gaithersburg, MD) containing 10% human AB serum, 50 U /mL penicillin, 50 µg/mL streptomycin, and 500 IU/mL interleukin-2 (IL-2) (unless otherwise specified). Bulk NK cells and NK cells isolated after limiting-dilution cloning were also grown in X-vivo 20 media (Cambrex Bio Science, Walkersville, MD) containing 10% heat inactivated human AB serum or 10% autologous serum.

*Flow cytometry:* Cell surface phenotype was determined by flow cytometry using the following antihuman monoclonal antibodies (mAbs): anti-CD16-phycoerythrin (PE) (clone B73.1 mouse immunoglobulin G1 [IgG1]; Becton Dickinson, San Jose, CA), anti-CD158a-PE (clone EB6, mouse IgG1; Immunotech, Marseilles, France), anti-CD158b-PE (clone GLT83, mouse IgG1; Immunotech), anti-CD56-Cy-chrome (clone MY31, mouse IgG1; Becton Dickinson), anti-CD3-fluorescein isothiocyanate (FITC) (clone SK7, mouse IgG1; Becton Dickinson), anti-LFA-1/CD11a-PE (clone HI111, mouse IgG1; PharMingen, San Diego, CA), and antihuman HLA-A, -B, -C-PE (clone G46-2.6, mouse IgG1; PharMingen). Cells were also stained with their corresponding isotype-matched control mAbs (Phar- Mingen). All samples were analyzed using EPICS XL-MCL (Beckman Coulter, Miami, FL). EXPO 32ADC software (Beckman Coulter) was used for data acquisition and analysis.

*Evaluation of NKG2 ligands on tumor cell lines:* Unconjugated antibodies to NKG2L, MICA (M363), MICB (M364), ULBP1 (M295), ULBP2 (M311) and ULBP3 (M551) (Amgen, Seattle, WA). Secondary antibody staining was accomplished with AlexaFluor488-conjugated goat antimouse immunoglobulin (Molecular Probes, Eugene, OR). Specific staining was revealed through the use of MAB272, an I-309 reactive IgG1 isotype control antibody (R&D Systems, Minneapolis, MN). Staining was evaluated using a FACScan instrument (Becton Dickinson).

*Isolation and expansion of autologous or allogeneic NK cells:* Mononuclear cells are first collected from cancer patients or healthy donors following a 5-25 liter apheresis procedure. The mononuclear fraction is then enriched for NK cells by negative depletion using one of 2 different immuno-magnetic bead strategies: 1) Dynal beads (Dynal NK cell isolation kit, Dynal Biotech, Lake Success, NY) according to the manufacturer's recommended conditions. 2) Using Miltenyi Biotec CliniMACS^{®} CD56 MicroBeads NK cell isolation Instrument following their cGMP guidelines. This involves a two step procedure comprising a T-cell depletion step using CliniMACS T cell CD3 MicroBeads and a subsequent positive selection of CD56⁺ NK cells.

Isolated NK cell are then expanded in vitro over 2-4 weeks in X-vivo 20 media (Cambrex Bio Science, Walkersville, MD) containing 10% heat inactivated human AB serum or 10% autologous serum and 500 IU/ml of IL-2 (IL-2 added every 4-7 days). One of 2 different irradiated feeder populations are added to bulk NK cells on day 1 and day 15: 1) Irradiated (75-100Gy) allogeneic EBV-LCL at a ratio of 10-100 EBVLCL cells (typically 20 EBVLCL cells) to every NK cell or 2) Irradiated (25-100Gy, typically 50Gy) autologous or allogeneic PBMCs at a ratio of 50-100 PBMCs (typically 100 PBMCs) to every NK cell. Using PBMC feeders, NK cells will expand by 2-3 logs over a 4 week period while EBVLCL feeders typically result in a 4-5 log expansion over the same time interval.

*Isolation and expansion of allogeneic KIR incompatible NK cells:* PBMCs obtained from healthy donors with HLA-C alleles homozygous in either group 1 (C-G1;ligates KIR2DL2/3 or group 2 (C-G2,ligates KIR2DL1) were enriched for NK cell populations by negative depletion using immunomagnetic beads (Dynal NK cell isolation kit, Dynal Biotech, Lake Success, NY) according to the manufacturer's recommended conditions. PBMC were incubated with an antibody mix containing anti-CD3, anti-CD14, anti-CD36, anti-CDw123, and anti-HLA class II DR/DP for 10 minutes at 4°C with gentle tilting and rotation. Cells were washed with PBS/0.1% human AB serum and centrifuged for 8 minutes at 500 g. The supernatant was discarded and the cell pellet was re-suspended in PBS/0.1 % human AB serum then incubated at 4° C for 10 minutes following the addition of depletion Dynabeads. The cell suspension was placed in a magnetic particle concentrator (Dynal MPC) for 2 minutes followed by removal of the supernatant and a repeat of the depletion bead step. NK cell purity was the determined by flow cytometry (typically in the 98% or higher range). One portion of the enriched NK cells was used to generate NK cell lines by limiting dilution at a density of 1-10 cells /well into a 96 well U-bottom plate containing 500 IU/ml of IL-2 and 10⁴ irradiated (100Gy) allogeneic EBV-LCL per well as feeder cells. Half of culture media was replaced weekly with new NK cell media containing fresh IL-2. The remaining portion of enriched NK cells was expanded as bulk NK populations in NK cell media containing 500 IU/ml of IL-2 and irradiated (100Gy) allogeneic EBV-LCL feeder cells (1000:1 EBV-LCL to NK cell ratio; 20:1 EBV-LCL to NK cell ratio was also used). An EBV-LCL to NK cell ratio ranging from about 1:1 to about 1000:1 can be effective in expanding NK cells. Two weeks following expansion, bulk NK cells were stained with anti-CD158b-FITC and anti-CD56 Cy-chrome and were then sorted using a MoFlo flow Cytometer (Dako-Cytomation Inc, Fort Collins, CO) to isolate CD56⁺/CD158b⁺ or CD56⁺/CD158⁺ populations which were re-expanded x 2 weeks using EBV-LCL feeders and NK cell media as above. In most experiments, allogeneic EBV-LCL with HLA-C alleles homozygous in a group opposite of the NK cells were used as feeder cells.

*Isolation and expansion of AKI-NK cells:* Peripheral blood mononuclear cells from cancer patients or healthy donors with HLA-C alleles homozygous in either group 1 (C-G1;ligates KIR2DL2/3) or group 2 (C-G2;ligates KIR2DL1) were enriched for NK cell populations by negative depletion using immunomagnetic beads (Dynal NK cell isolation kit, Dynal Biotech, Lake Success, NY) according to the manufacturer's recommended conditions. Briefly, PBMC were incubated with an antibody mix containing anti-CD3, anti-CD14, anti-CD36, anti-CDw123, and anti-HLA class II DR/DP for 10 minutes at 4°C with gentle tilting and rotation. Cells were washed with PBS/0.1% human AB serum and centrifuged for 8 minutes at 500 g. The supernatant was discarded and the cell pellet was re-suspended in PBS/0.1 % human AB serum then incubated at 4° C for 10 minutes following the addition of depletion Dynabeads. The cell suspension was placed in a magnetic particle concentrator (Dynal MPC) for 2 minutes followed by removal of the supernatant and a repeat of the depletion bead step. NK cells were then stained with anti-CD158b-FITC, anti-CD158a-PE, anti-CD3-PerCP, and anti-NKB1-APC. Using a MoFlo flow cell sorter, NK cells were sorted into 5 distinct NK subpopulations: CD3 negative NK cells (bulk NK), CD158b-/CD158a-/NKB1-/CD3- NK cells, CD158b+/CD158a-/NKB1-/CD3- NK cells and CD158a+/CD158b-/NKB1-/CD3- NK cells and NKB1+/CD158b-/CD158a-/CD3- NK cells. Alternatively, NK cells can be sorted by antibody conjugated magnetic beads, e.g., anti-CD56, anti-CD158a, or anti-CD158b, and NK cells bound to beads are enriched on a magnetic column. The magnetic beads can also be used with a secondary antibody binding to the anti-CD56, anti-CD158a, or anti-CD158b magnetic beads. Sorted cells were then either directly expanded using irradiated EBV-LCL as stimulators (1000:1 EBV-LCL to NK cell ratio) or were cloned by limiting dilution in 96-well round bottom cell culture plates containing 200 uL ofNK cell culture media containing 500 IU/ml of IL-2 and 10⁴ irradiated (100Gy) allogeneic EBV-LCL per well as feeder cells.

NK bulk populations, NK flow sorted subpopulations, and NK clones were expanded for 4-6 weeks using EBV-LCL feeders and NK cell media as above. NK populations were then re-phenotyped and any T cell populations representing greater than 2% of the total population were depleted by Dynal CD3- one-step magnetic bead depletion. Any significant presence of other KIR-expressing NK cells were depleted either by flow-sorting or using a two-step Dynal magnetic bead depletion technique.

*Optimizing NK cell expansion:* In bulk NK cell expansions, EBV-LCLs heterogeneous or homogeneous for C-G1 or C-G2 were used as feeders, with the proportions of expanded CD158a⁻ or CD158b- NK cells determined by flow cytometry. An EBV-LCL to NK cell ratio ranging from about 1:1 to about 1000:1 1 can be used in expanding NK cells. Fold expansion was measured by staining bulk NK cells with trypan blue, then dividing the total number of viable cells after expansion by the number of viable cells before expansion.

*Cytotoxicity assays:* Following NK cell expansions, NK cells then were tested for cytotoxicity against autologous and allogeneic KIR matched and KIR mismatched EBV-LCL and solid tumor lines. Assessment of NK cell cytotoxicity was determined using standard chromium release assays. Takahashi, et al., Blood, 103: 1383-1390, 2004. Briefly, 10⁶ target cells were incubated with 100 µCi of Na₂⁵¹CrO₄ (Amersham Biosciences, NJ) for 1 h, washed and then re-suspended at a concentration of 0.7 - 1 x 10⁴ cells/100 uL. One hundred uL each of effector (effector:target ratio of 10-20:1) and target (in replicates of 2 or 3) were co-cultured in 96-well round-bottom plates (Nalge Nunc International, NY) for a total volume of 200 ul/well. After a 5-hour incubation at 37 C°,100 ul of supernatant was harvested and its radioactive content was measured using a gamma counter.

The percentage of specific lysis was calculated using the formula: 100 x (cpm released from test sample - cpm spontaneous release)/(cpm maximum release - cpm spontaneous release). All values shown represent the average of duplicates +/- one standard deviation. Some targets were preincubated with 20 µg/mL of the monoclonal antibody W6/32 (anti-pan class 1 MHC monoclonal antibody; DAKO) to assess the impact of MHC class I blockade on NK cell cytotoxicity.

### EXAMPLE 2

### Expansion of NK cells on an EBVLCL feeder cell line

NK cells containing EBVLCL feeders could be expanded more than 10⁴-fold, significantly higher compared to NK cell populations cultured in IL-2 containing SCGM media alone, where approximately a 10²-fold net expansion was typically observed. See Table 2. A method was developed to expand a high percentage of KIR 2DL2/3 positive NK cells. A significantly higher percentage of KIR 2DL2/3 positive NK cells can be expanded using EBVLCL feeders that either lack or have heterozygous expression for HLA-C alleles known to ligate KIR2DL2/3.

Preferential proliferation of NK cells occurs when EBV-LCLs are cultured with PBMCs. Perussia, et al., Nat. Immun. Cell Growth Regul., 6: 171-188, 1987; Mainiero, et al., J. Immunol., 152: 446-454, 1994. Therefore, it was investigated whether EBV-LCL feeder cells could be used to enhance the expansion of bulk NK cell populations. NK cells (1 X 10³) enriched from PBMCs by negative depletion (typically 90% or more CD3⁻ and CD56⁻) were cultured in stem cell growth medium (SCGM) containing 500 IU of IL-2, alone or with 1 X 10⁶ irradiated EBV-LCLs. After 14 days, NK cells containing EBV-LCL feeders expanded more than 10⁴-fold compared with NK cell populations cultured in IL-2 containing SCGM media alone, where approximately a 10²-fold net expansion was typically observed. No significant differences in NK cell expansion were observed when using EBV-LCLs with HLA-C alleles that were matched compared with mismatched for NK cell HLA-C groups. However, a significantly higher percentage of KIR 2DL2/3-positive NK cells was expanded from an HLA-C group 1 homozygous donor when EBV-LCL feeders were used that either lacked or had heterozygous expression for HLA-C alleles known to ligate KIR2DL2/3 (Table 2). Varying the IL-2 concentration from 5 to 500 IU/mL did not influence NK cell expansion when EBV-LCLs were used as feeders.

**Table 2: Expansion of bulk NK cells: Donor HLA-C group 1 homozygous**

| **HLA-Cw Locus of Feeder Cells** | **Expansion Fold** | **CD3-CD56+ or CD16+** | **CD3-CD56+CD158b+** |
|---|---|---|---|
| Homozygous for C-G2 | (1.8±1.6)x10⁴ | 79.5 ± 5.2% | 41 ± 9.1%* |
| Homozygous for C-G1 | (1.32 ± 0.87) x 10⁴ | 72.5 ± 11.6% | 24 ± 8.8%* |
| Autologous | | | |
| Heterozygous for C-G2 and C-G1 | (1.8±0.45)x10⁴ | 78.6 ± 4.5% | 33 ± 9.5% |

| **IL-2 Concentration (IU/ml)** ** | | | |
|---|---|---|---|
| 100 | 2.6 x 10⁴ | 76.6% | 35.5% |
| 50 | 2.5 x 10⁴ | 75.7% | 27.2% |
| 5 | 6.8 x 10³ | 68.2% | 21.8% |

| | | | |
|---|---|---|---|
| NK cells were enriched by negative selection from PBMC of a healthy donor (KR0350) homozygous for HLA-C group 1 (C-G1), then expanded in vitro using irradiated EBVLCL feeders: 1)HLA-C group 2 (C-G2) EBVLCL; 2) Autologous EBVLCL; 3) HLA-C group is heterozygous EBVLCL. Two weeks later, expanded NK cells were counted and phenotype analysis was performed. The difference in CD3-CD56+CD158b+ positive population between homozygous for C-G2 feeder cells and autologous feeder cells was significant, *p=0.0085. Values represent averages ±SD from three independent experiments. ** Values represent averages from two independent experiments. | | | |

### EXAMPLE 3

### Allogeneic NK-cells more cytotoxic to tumor targets mismatched for KIR-ligands than KIR-ligand matched counterparts

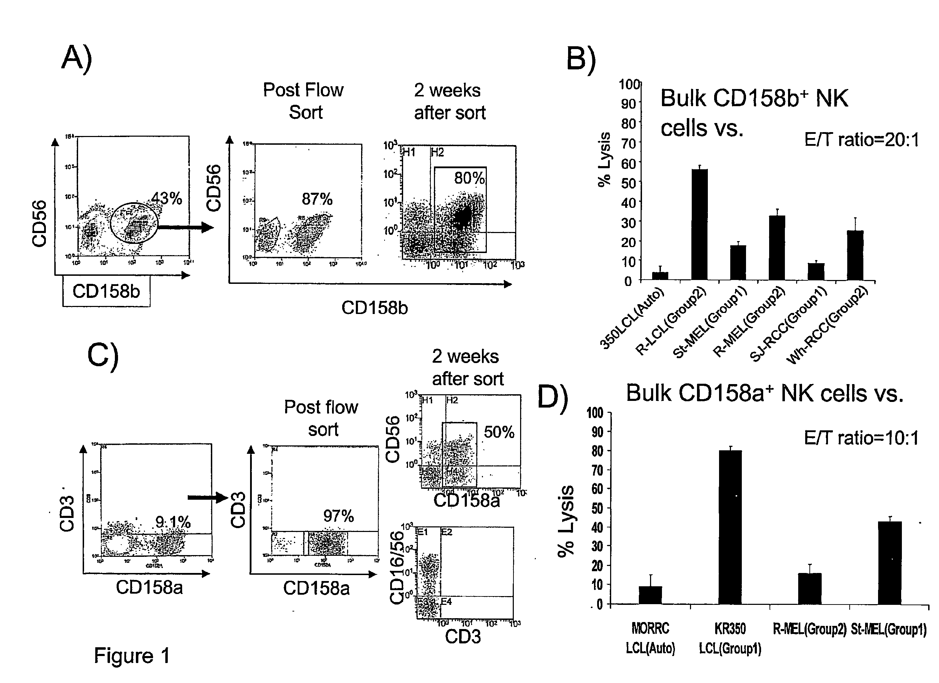

Allogeneic NK-cells were more cytotoxic to tumor targets mismatched for KIR-ligands than their KIR-ligand matched counterparts. An assessment was first performed *in vitro* to determine whether melanoma and RCC cells would be more susceptible to killing by bulk allogeneic NK populations when the tumor lacked an HLA-C allele for a specific KIR expressed on NK cell effectors. NK cells enriched from PBMCs by negative selection from a donor homozygous for HLA-C group 1 (C-G1) were expanded in vitro using irradiated EBV-LCLs as feeder cells. Two weeks later, NK cells expressing CD158b (KIR 2DL2/3) were isolated by flow sorting and were then expanded for 2 more weeks to remove remaining bound antibodies (Figure 1A). KIR ligand compatible (*e.g.*, homozygous for C-G1) or KIR ligand incompatible (eg, homozygous for C-G2) tumor targets were then tested in vitro for differences in susceptibility to the cytotoxic effects of CD158b-enriched (approximately 80% positive) bulk NK cells.

A significantly greater percentage of KIR incompatible, C-G2 homozygous, melanoma, and RCC lines were lysed by CD158benriched NK cells than by KIR compatible C-G1 homozygous tumor counterparts (Figure 1B). A similar pattern of cytotoxicity was observed against KIR ligand-compatible (autologous) compared with KIR ligand-incompatible EBV-LCL targets. Likewise, CD158a-enriched NK cells lysed a higher percentage of KIR incompatible, C-G1 homozygous EBV-LCL and melanoma cells compared with C-G2 homozygous KIR-matched targets (Figures 1B,D).

Figure 1 shows the isolation of CD 158b (KIR 2DL2/3) and CD 158a (KIR 2DL1) positive NK populations. In Figure 1A, NK cells were enriched by negative selection from PBMC of a healthy donor (KR0350) homozygous for HLA-C group 1 (C-G1), then expanded *in vitro* using irradiated EBVLCL feeders. Two weeks later, CD3-/CD56+/CD 158b+ NK cells were isolated by flow sorting, then expanded an additional 2 weeks to remove any remaining surface bound antibodies. In Figure 1B, at a 20:1 effector to target ratio, NK cells enriched for CD 158b (80% positive) lysed a significantly higher percentage of KIR incompatible EBVLCL, melanoma and RCC cells homozygous for HLA-C group 2 (KIR incompatible) compared to HLA-C group 1 homozygous KIR matched tumor targets. In Figure 1C, CD158a+ NK cell populations were isolated by flow sorting from healthy donor MORRC homozygous for HLA-C group 2 (C-G2). In Figure 1D, these cells lysed a significantly higher percentage of EBVLCL and melanoma cells homozygous for HLA-C group 1 (KIR incompatible) compared to HLA-C group 2 homozygous KIR matched targets.

NK cell lines were isolated from healthy donors or patients with cancer that specifically killed KIR-incompatible EBV-LCL populations and then evaluated their cytotoxic effects against KIR ligand-matched compared with -mismatched melanoma or RCC targets. PBMCs from healthy donors or cancer patients homozygous for C-G1 or C-G2 were enriched for NK cell populations by negative depletion, then cloned by limiting dilution using allogeneic KIR ligand-mismatched EBV-LCLs as feeder cells. NK lines were phenotyped using fluorescence-activated cell sorter (FACS) and were tested for cytotoxicity against KIR-matched (autologous) or KIR-mismatched EBV-LCL targets.

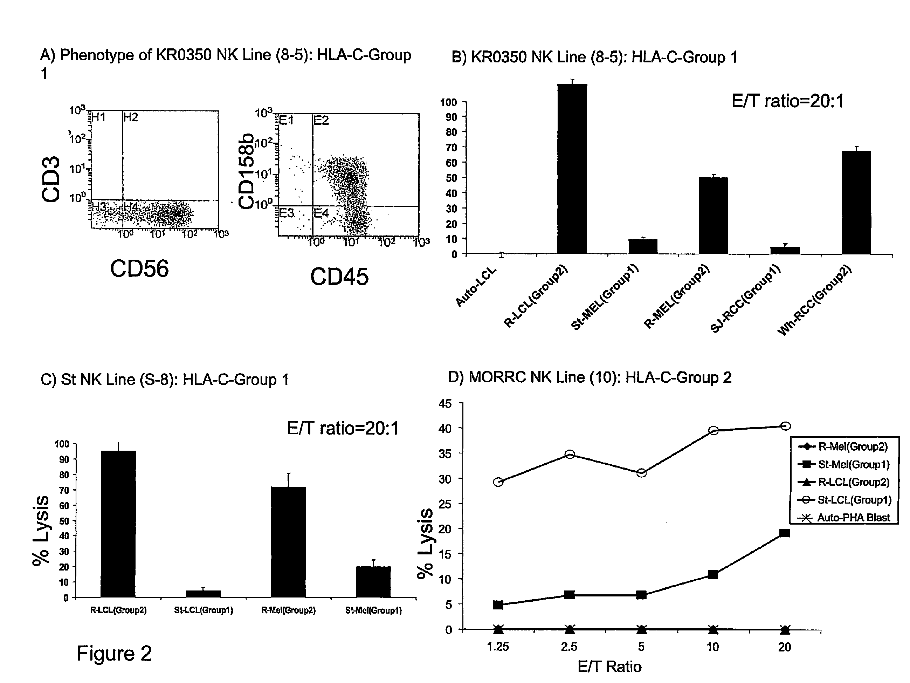

Thirty-four CD3-/CD56- NK lines were expanded from a healthy donor homozygous for C-G1 (KR0350); 9 lysed EBV-LCL cells homozygous for C-G2 (35%-100% lysis at an effector-target [E/T] ratio of 20:1), with minimal to no lysis of KIR-matched (autologous) EBV-LCLs(data not shown). Line 8-5 expanded sufficiently for further experiments (Figure 2A).

Although this line had no cytotoxicity against autologous EBV-LCLs, it lysed nearly 100% of EBV-LCLs homozygous for C-G2, consistent with killing through KIR incompatibility. At a 20:1 E/T ratio, a significantly higher percentage of KIR ligand-mismatched melanoma and RCC cells (homozygous for C-G2) were killed than KIR ligand-matched (homozygous for C-G1) tumor targets (Figure 2B). Similarly, enriched NK cells were expanded after limiting dilution from the PBMCs of a patient (St) with widely metastatic melanoma who was homozygous for C-G1; NK line S-8 from this patient killed a significantly higher percentage of KIR ligand-mismatched EBV-LCLs (95%) and melanoma cells (72%) than KIR ligand-matched autologous targets (5% and 19% of EBV-LCL and melanoma cells respectively; Figure 2C).

NK lines could also be expanded from a healthy donor homozygous for C-G2 with enhanced cytotoxicity against KIR ligand-mismatched allogeneic EBV-LCLs and melanoma cells homozygous for C-G1 compared with KIR ligand-matched allogeneic EBV-LCLs and melanoma cells homozygous for C-G2 (Figure 2D).

Figure 2 shows that RCC and melanoma cells are more susceptible to the cytotoxic effects of KIR-incompatible NK clones than KIR-matched lines. NK cells were enriched and then cloned by limiting dilution from the PBMCs of cancer patient St (homozygous for HLA-C group 2) or from healthy donors homozygous for HLA-C group 1. (A) NK cell line 8-5, expanded from a C-G1 homozygous healthy donor (KR0350), killed a significantly higher percentage of C-G2 homozygous KIR-incompatible EBV-LCL, melanoma, and RCC cells than KIR-matched autologous EBV-LCL or KIR-matched allogeneic C-G1 homozygous melanoma and RCC cells (B). (C) NK line S-8, expanded from a C-G1 homozygous patient with widely metastatic melanoma (St), killed a significantly higher percentage of K1R-mismatched C-G2 homozygous EBVLCLs and melanoma cells than autologous KIR-matched EBV-LCL and melanoma cells, in which minimal cytotoxicity was observed. Mean values +/-SD of 3 samples are shown. (D) MORRC NK line 10, expanded from a C-G2 homozygous healthy donor (MORRC), was cytotoxic to KIR-incompatible C-G1 homozygous but not KIR-matched C-G2 homozygous targets.

MHC class I blockade has been shown to enhance NK cell cytotoxicity against KIR ligand-matched tumors in vitro. Therefore, the cytotoxic potential of allogeneic KIR ligand-mismatched NK cells was investigated against RCC and melanoma cells compared with KIR ligand-matched NK cells targeting tumors in which MHC class I had been blocked with the monoclonal antibody W6/32.

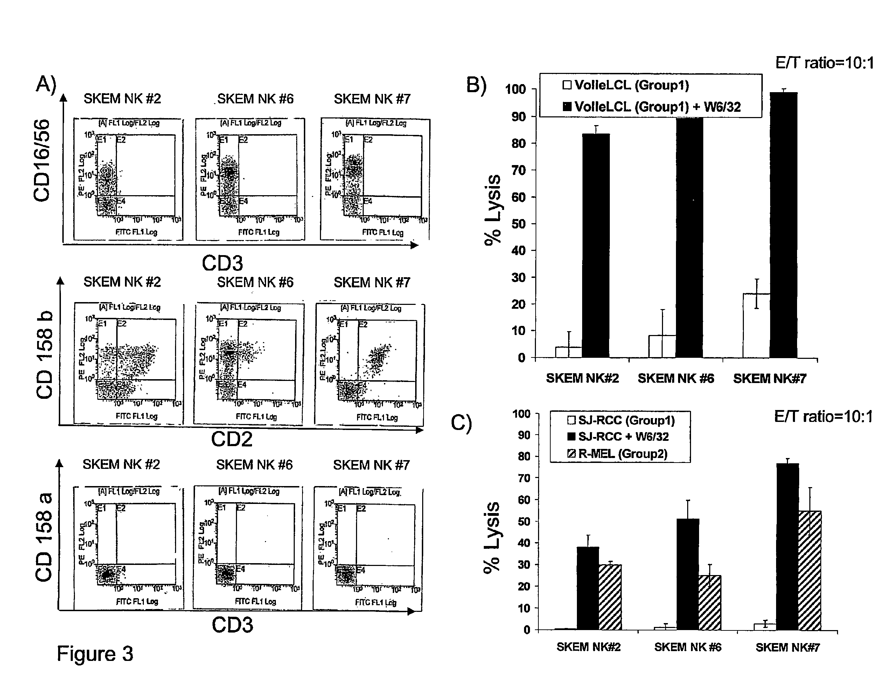

NK cells enriched from the PBMCs of a healthy donor, SKEM (C-G1 homozygous; HLA-identical sibling to RCC patient SJ) were expanded after limiting dilution and screened for cytotoxicity against KIR-matched (C-G1 homozygous) versus KIR-mismatched (C-G2 homozygous) targets. Three NK lines (SKEM NK lines 2, 6, and 7; Figure 3A) with significantly greater cytotoxicity against C-G2-mismatched versus C-G1-matched EBV-LCLs were identified.

At a 10:1 E/T ratio, NK lines lysed 4% to 25% of Volle EBV-LCLs (matched for C-G1); the cytotoxicity of all 3 lines increased significantly (greater than 80%) when Volle EBV-LCLs were precultured with the monoclonal antibody W6/32 (pan class 1 MHC antibody), consistent with MHC class I molecules on the target cells functioning as NK cell KIR ligands (Figure 3B). SKEM NK lines were significantly more cytotoxic against KIR ligand-mismatched, C-G2 homozygous R-melanoma cells than against KIR ligand-matched, C-G1 homozygous SJ RCC cells, in which virtually no cytotoxicity was observed. Preculturing SJ RCC cells with the pan MHC class I monoclonal antibody W6/32 substantially increased the cytotoxic effects of all 3 NK cell lines against this KIR-matched tumor target.

Figure 3 shows allogeneic KIR incompatible NK cell lines isolated by limiting dilution cloning have enhanced cytotoxicity against KIR-mismatched tumor targets. In Figure 3A, NK cell lines 2, 6, and 7, were isolated by limiting dilution from the PBMC of a healthy donor SKEM (C-G1 homozygous: HLA identical sibling to RCC patient SJ). In Figure 3B, all 3 lines lysed a minority of C-G1 homozygous KIR matched VOLLE EBVLCL. Pre-incubation of VOLLE EBVLCL with W6/32 significantly enhanced the cytotoxic effects of all 3 lines, consistent with MHC class I molecules inhibiting NK cell function. In Figure 3C, NK Lines 2, 6, and 7 were significantly more cytotoxic to KIR ligand mismatched R-MEL cells (C-G2 homozygous) compared to KIR matched (C-G1 homozygous) SJ cells, where virtually no cytotoxicity was observed. Pre-incubation of SJ RCC cells with W6/32 significantly increased the cytotoxic effects of all 3 lines against this KIR matched RCC target.

### EXAMPLE 4

### Gamma irradiated NK cell maintain cytotoxicity against tumor cells for at least 48 hours following irradiation.

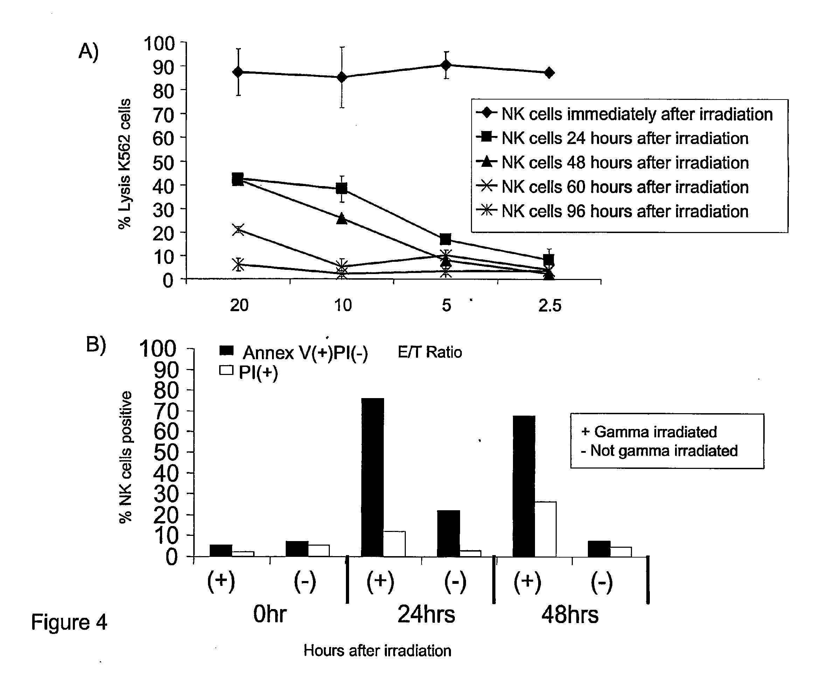

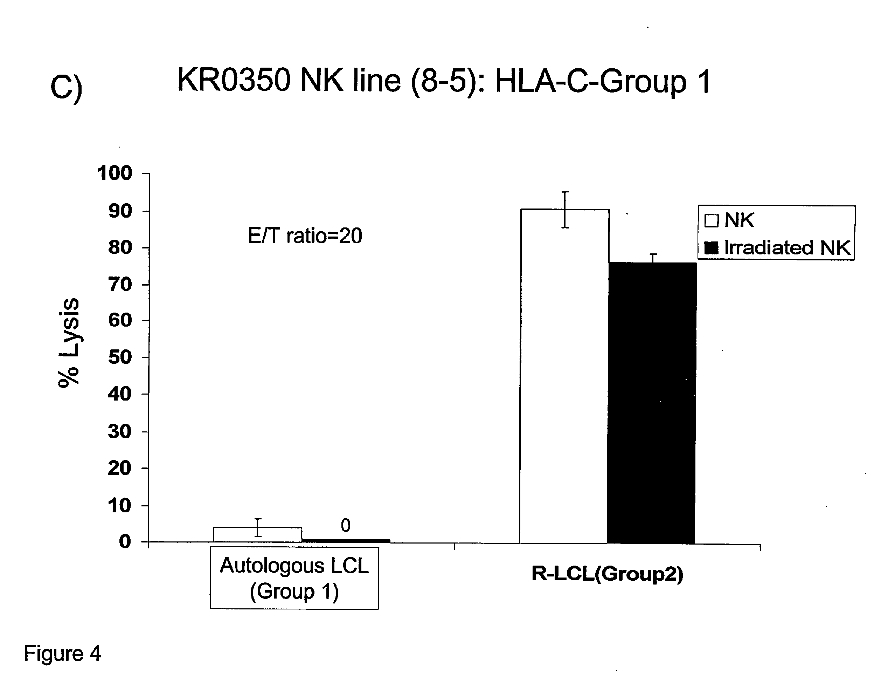

*In vitro* data suggest adoptively infused KIR-incompatible allogeneic NK cells might also have beneficial antitumor effects in vivo. Therefore, the impact of gamma irradiation on NK cell-mediated tumor cytotoxicity was further investigated. Bulk NK cells expanded from PBMCs of healthy donors were gamma irradiated (50 Gy) and then tested for cytotoxicity against NK cell-sensitive K562 cells 0, 24, 48, 60, and 96 hours after irradiation (Figure 4A). Although most NK cells quickly became annexin V- or propidium iodide (PI)-positive (Figure 4B) by FACS (consistent with radiation-induced apoptosis and death), significant cytotoxicity against K562 cells could still be measured 48 hours after irradiation. NK cell line 8-5 from healthy donor KR0350 maintained its ability to kill KIR-incompatible EBV-LCL cells 24 hours after irradiation, with only a slight decrease in its cytotoxic capacity compared with a nonirradiated control (Figure 4C).

Gamma irradiated NK cells retain significant cytotoxicity against tumor targets up to 48 hours following irradiation. Furthermore, they preserve their ability kill KIR incompatible tumor targets for at least 24 hours following irradiation. Figure 4 shows gamma irradiated NK cell maintain cytotoxicity against tumor cells for at least 48 hours following irradiation. Bulk NK cells expanded from the PBMC of healthy donor KR0350 were gamma irradiated (50 Gy) then tested for cytotoxicity against K562 cells Figure 4A shows percent lysis of K562 tumor cells by allogeneic NK cells at 0, 24, 48, 60 and 96 hours following gamma irradiation of NK cells. Figure 4B shows that although irradiation induced NK apoptosis (annexin V positive) and necrosis (propidium iodide positive), persistent NK cell cytotoxicity was observed up to 48 hours following gamma irradiation. Figure 4C shows that C-G1 homozygous NK cell lines (8-5) from donor KR0350 maintained a high level of cytotoxicity against KIR incompatible R-EBVLCL cells 24 hours following 50 Gy of gamma irradiation.

### EXAMPLE 5

### Identification and expansion of a subdominant NK cell fraction with enhanced cytotoxicity against autologous tumor cells

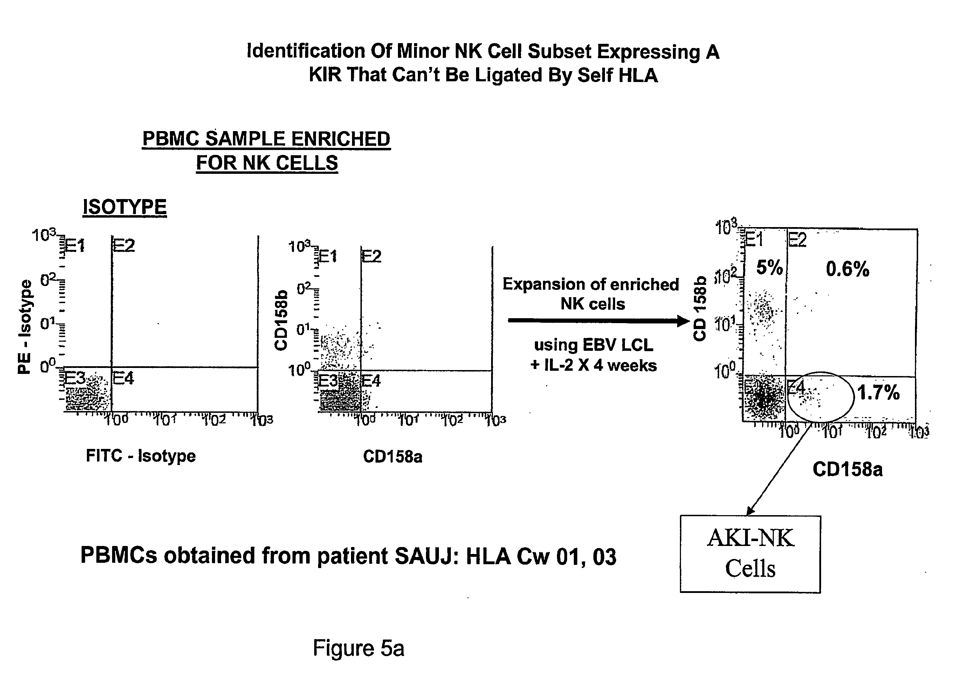

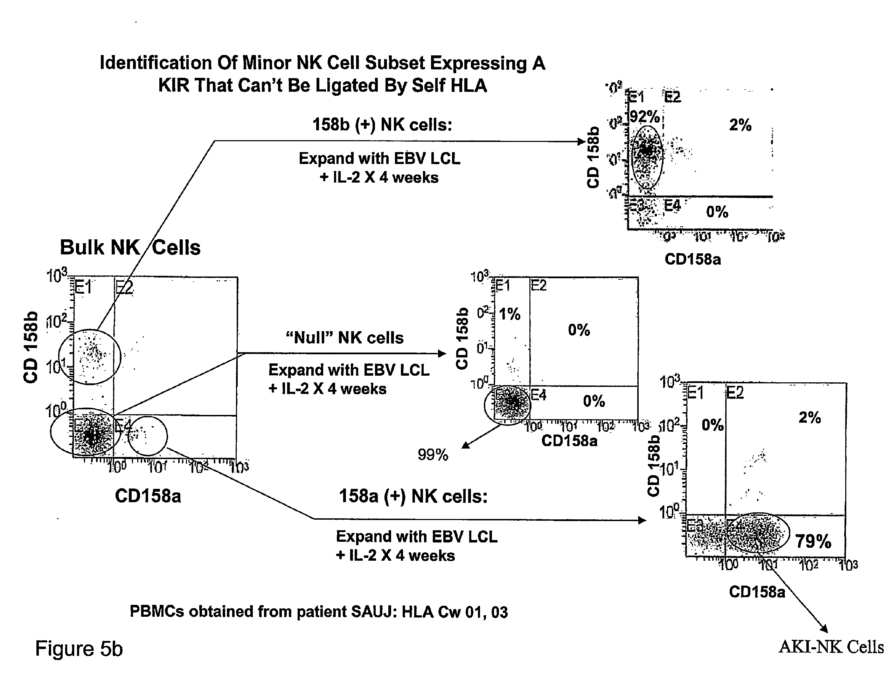

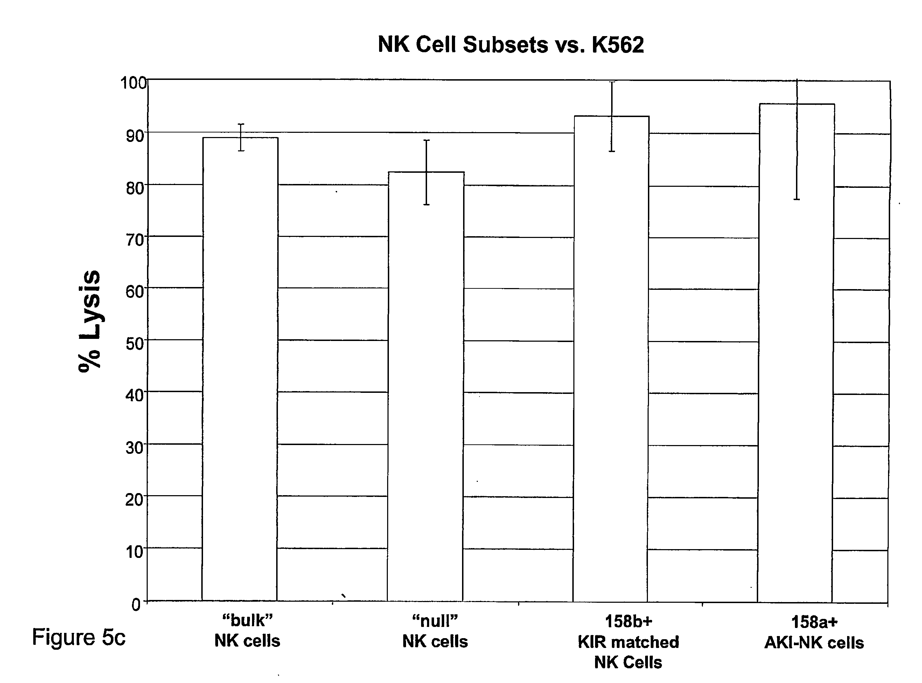

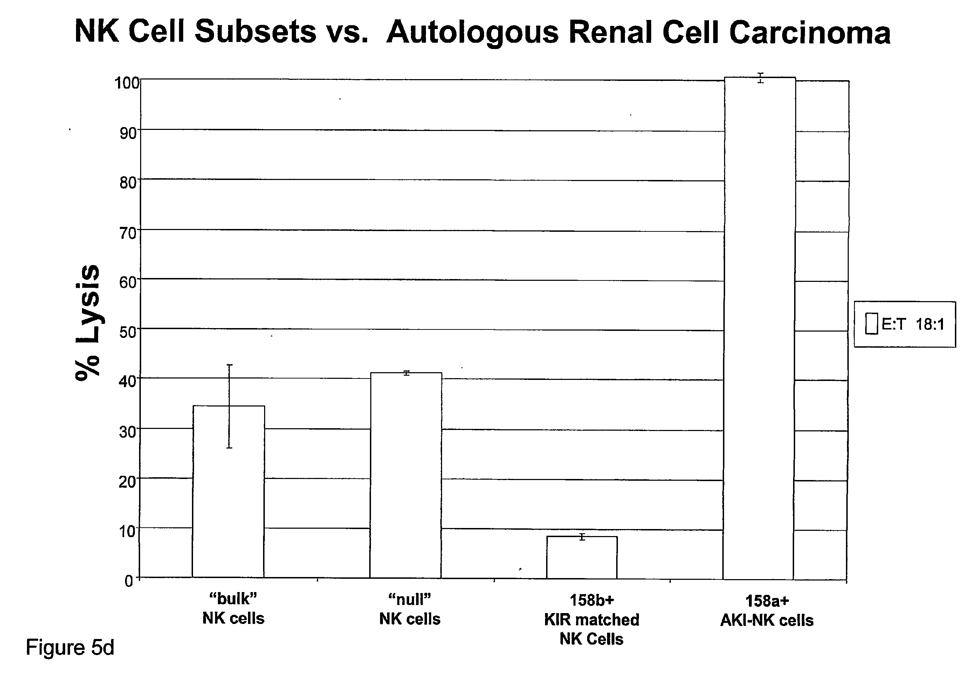

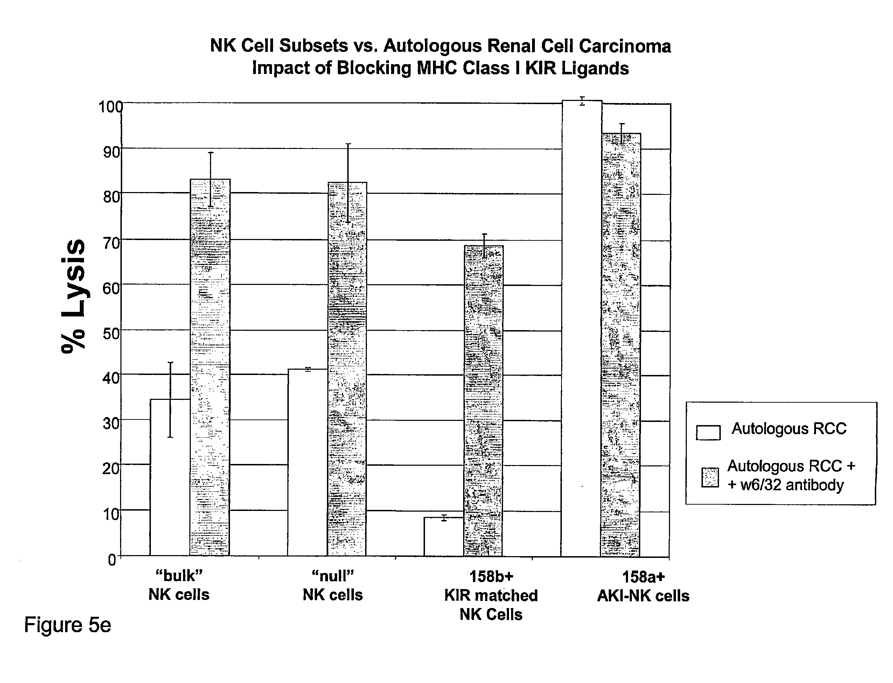

Subdominant NK cell populations expressing KIR molecules that cannot be ligated by self-HLA molecules (AKI-NK cells) can be isolated and expanded *in vitro* and are significantly more cytotoxic to autologous tumor targets compared to unselected "bulk" NK cells. See Figure 5.

Figure 5 shows the identification and expansion of a subdominant NK cell fraction with enhanced cytotoxicity against autologous tumor cells. Figure 5A shows that PBMCs collected from a patient homozygous for group 1 KIR ligands (Cw 01, 03) were enriched for NK cells by negative depletion using immunomagnetic beads (Dynal NK cell isolation kit). NK cells were expanded over 2 weeks using irradiated EBVLCL feeder cells. A subdominant fraction (1.7%) of autologous KIR incompatible NK cells (AKI-NK) that were CD158a+/CD158b- NK cells was identified. Figure 5B shows that NK cells were either expanded in bulk ("bulk NK" cells) or were sorted using FACS into one of three different subpopulations 1) CD158b+/CD158a- KIR matched NK cells 2) CD158b-/CD158a- ("Null NK" cells) 3) CD158b-/CD158a+ (AKI-NK) NK cells. Sorted NK cell populations were then expanded x 2 weeks *in vitro* using EBV-LCL feeder cells. Figure 5C shows that all 4 different NK cell fractions were killed K562 cells. Figure 5D shows that AKI-NK cells demonstrated enhanced cytotoxicity against autologous tumor cells (Renal cell carcinoma-RCC) compared to bulk NK cells, CD158b+/CD158a- KIR matched NK cells and null NK cells).

### EXAMPLE 6

### RNA interference (RNAi) post-transcriptional gene silencing (PTGS) to knock out expression and function of inhibitory KIR

Based on these results, RNA interference (RNAi) post-transcriptional gene silencing (PTGS) is used to knock out expression and function of inhibitory KIR, thus enhancing NK cell cytotoxicity against tumor cells. A lentiviral vector encoding an RNAi is being developed that will specifically knock down the NK cell's inhibitory KIR long cytoplasmic domain. The RNAi will be specific for the long cytoplasmic tails of inhibitory KIRs 2DL2/3 and 2DL1.

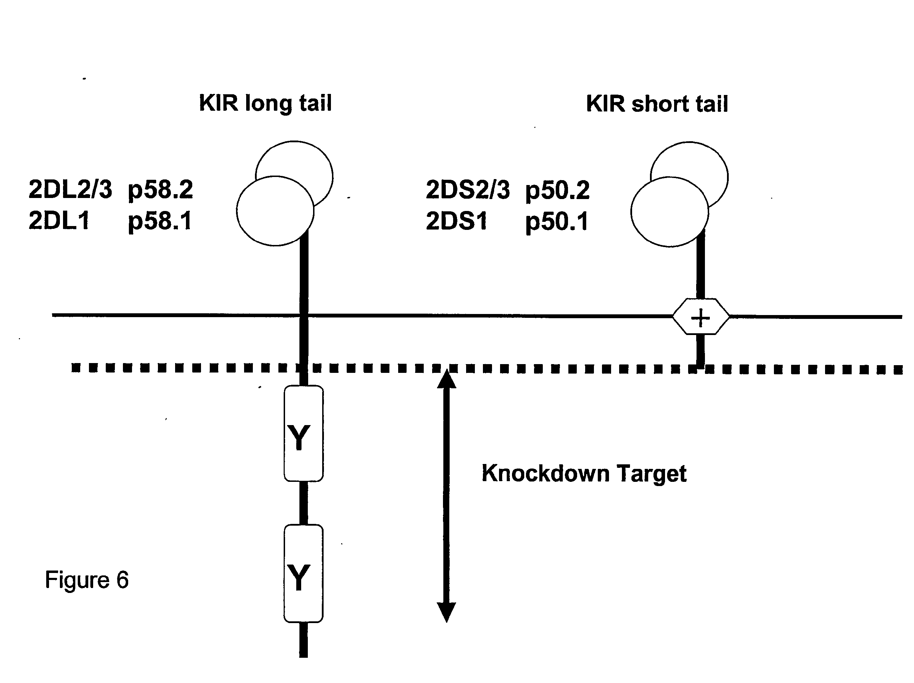

Figure 6 shows a proposed experiment to demonstrate enhancement of NK cell antitumor effects by lentiviral transduction with RNAi that will act to knock down the inhibitory KIR long cytoplasmic domain. Bulk NK populations will be enriched for by either negative depletion or positive selection. Bulk NK cells will be transduced with a lentiviral vector encoding an RNAi specific for the long cytoplasmic tails of inhibitory KIRs 2DL2/3 and 2DL1. It is anticipated such transduced NK cells will have enhanced cytotoxicity against tumor cells compared to wild-type NK cell populations.

The proposed experiment transduces autologous NK cells with this lentiviral vector. RNAi transduction will disrupt the ability of SHP-1 (a tyrosine phosphatase) to bind to the ITIM, thus preventing NK cell inactivation through the inhibitory KIR. Importantly, the short cytoplasmic tail of the complimentary activating KIR (*i.e*., 2DS2/3 and 2DS 1) will remain intact and capable of triggering a downstream NK cell activation cascade. NK cells that have been transduced to inactivate inhibitory KIR function are anticipated to have enhanced cytotoxicity against autologous and allogeneic tumor targets. It is further anticipated that such a population, when adoptively infused, will induce regression of metastatic solid tumors and hematological malignancies.

### EXAMPLE 7

It has been demonstrated that similar NK cell-mediated effects occur *in vitro* against neoplastic cells of epithelial origin. Specifically, melanoma and RCC cells are more susceptible to the cytotoxic effects of allogeneic NK cells expressing KIR that cannot be ligated by the tumor's inhibitory HLA-C alleles because of HLA mismatching. NK cell lines were generated from donors or patients who were homozygous for group 1 or group 2 HLA-C alleles, in whom the frequency of NK cells with alloreactivity against targets lacking the same HLA-C allele group was high. Furthermore, EBV-LCL, melanoma, and RCC tumor targets that were homozygous for group 1 or group 2 HLA-C alleles were used, so that the impact of KIR mismatching on NK cell cytotoxicity could be evaluated.

Although they varied in frequency, NK cells that selectively killed HLA-C group-mismatched EBV-LCLs could be isolated from all donors studied. In most cases, NK cells with enhanced cytotoxicity against KIR-incompatible EBV-LCLs also killed a significantly higher percentage of melanoma and RCC cells that lacked a matching inhibitory HLA-C KIR ligand compared with KIR ligand-matched tumor cells. The differences in the level of cytotoxicity were often striking, with substantially higher (more than 60%) lysis of KIR ligand-mismatched tumor targets compared with KIR-matched targets in which little to no lysis occurred. This NK cell phenomenon was not restricted to healthy donors; NK cell lines from cancer patients homozygous for C-G1 or C-G2 were identified that failed kill autologous tumor cells while having significantly greater cytotoxicity against allogeneic KIR ligand- mismatched tumor lines. KIR incompatibility was restricted to the HLA-C locus. One can speculate that such NK cell populations with KIR mismatching was not evaluated in HLA-B or -A loci can have enhanced cytotoxicity similar to that shown to occur against AML and EBV-LCL cells.

Table 4 shows HLA-C and HLA-B loci of an enriched autologous NK cell population. HLA-C Group 1 Cw alleles share Ser77 and Asn80 in the α1 helix and ligate KIR2DL2/3 (CD158b). HLA-C Group 2 Cw alleles share Asn77 and Lys80 in the α1 helix and ligate KIR2DL1 (CD158a). HLA-B loci that fall into "Bw4" group will ligate NKB1 KIR. HLA-B loci that fall into "Bw6" group do not ligate NKBI KIR.

**Table 4: HLA loci of enriched autologous NK cell populations**

| **HLA-C** | | **HLA-B** | |
|---|---|---|---|
| *Group 1 Cw alleles* These HLA-C loci share Ser77 & Asn80 in α1 helix, ligate KIR2DL2/3 (CD158b) | *Group 2 Cw alleles* These HLA-C loci share Asn77 & Lys80 in α1 helix, ligate KIR2DL1 (CD158a) | HLA-B loci that fall into "Bw4" group ligate NKB1 KIR. | HLA-B loci that fall into "Bw6" group do not ligate NKBI KIR |
| | | Bw-5 | Bw-7 |
| | | Bw-13 | Bw- 8 |
| | Cw2 (all) | Bw-1513 | Bw-12 |
| | Cw0307, 0315 | Bw-1516 | Bw- 14 |
| Cw1 (all) | Cw4 (all) | Bw-1517 | Bw- 18 |
| Cw3 (all except0307, 0310,0315) | Cw5 (all) | Bw-1523 | Bw- 2708 |
| Cw 7 (all except 0707, 0709) | Cw6 (all) | Bw-1524 | Bw- 35 |
| Cw 8 (all) | Cw0707, 0709 | Bw-17 | Bw- 39 |
| Cw12(all 1205, 12041,except 12042) | Cw1205, 12041, 12042 | Bw-21 | Bw- 40 |
| Cw13 (all) | Cw15 (all except 1507) | Bw-27 | Bw- 4005 |
| Cw14 (all except 1404) | Cw1602 | Bw-37 | Bw- 41 |
| Cw1507 | Cw17 (all) | Bw-38 | Bw- 42 |
| Cw16 (all except 1602) | Cw18 (all) | Bw-44 | Bw- 45 |
| | | Bw-47 | Bw- 46 |
| | | Bw-49 | Bw- 48 |
| | | Bw-51 | Bw- 50 |
| | | Bw-52 | Bw- 54 |
| | | Bw-53 | Bw- 55 |
| | | Bw-57 | Bw- 56 |
| | | Bw-58 | Bw- 60 |
| | | Bw-59 | Bw- 61 |
| | | Bw-63 | Bw- 62 |
| | | Bw-77 | Bw- 64 |
| | | | Bw- 65 |
| | | | Bw- 67 |
| | | | Bw- 71 |
| | | | Bw- 72 |
| | | | Bw- 73 |
| | | | Bw- 75 |
| | | | Bw- 76 |
| | | | Bw- 78 |
| | | | Bw- 81 |

The balance between activating and inhibitory signals ultimately determines whether target cells will be susceptible to NK cell-mediated lysis. The presence of LFA-1 on myelogenous leukemia (AML and CML) cells and its absence on NK cell-resistant ALL cells suggested this adhesion molecule might function as a ligand for NK cell-activating receptors. The activating ligands on tumor cells used in these experiments that were necessary to initiate alloreactive NK cell killing are being investigated. Flow cytometric analysis showed all RCC and melanoma lines used in this study to be LFA-1 negative. However, tumor lines used in this study had variable densities of MHC class I chain-related antigens (MICA or MICB) (Bauer, et al., Science, 285: 727-729, 1999; Wu, et al., Science, 285: 730-732, 1999) or UL16-binding proteins (Cosman, et al., Immunity, 7: 273-282, 1997; Cosman, et al., Immunity, 14: 123-133, 2001; Sutherland, et al., Immunol. Rev., 181: 185-192, 2001; Sutherland, et al., J. Immunol., 168: 671-679, 2002)(ULBPs; See Table 5), known ligands for the NK cell-activating receptor NKG2D. Ruggeri, et al., Blood, 94: 333-339, 1999.

**Table 5. Expression of NKG2D ligands measured by flow cytometry**

| NKG2D ligands | Tumor cell lines, % | | | |
|---|---|---|---|---|
| | St-MEL | R-MEL | SJ-RCC | Wh-RCC |
| MIC A/B | 8 | 13 | 17 | 10 |
| ULBP1 | 0 | 0 | 52 | 0 |
| ULBP2 | 0 | 0 | 52 | 0 |
| ULBP3 | 80 | 0 | 98 | 9 |

| | | | | |
|---|---|---|---|---|
| Expression of NKG2D ligands; stress-inducible surface glycoprotein MIC A/B, UL 16-binding protein (ULBP), ULBP1, ULBP 2, and ULBP 3 were measured using flow cytometry. | | | | |

Studies have shown that leukemia, lymphoma, (Salih, et al., Blood, 102: 1389-1396, 2003) and melanoma lines (Pende, et al., Eur. J. Immunol., 31: 1076-1086, 2001; Pende, et al., Cancer Res., 62: 6178-6186, 2002) with these ligands can be specifically lysed by NKG2D-expressing NK cells. It remains to be determined whether the level of expression of different activating ligands might be used as a surrogate to identify those malignancies with susceptibility to KIR-incompatible NK cells. Natarajan, et al., Annu. Rev. Immunol., 20: 853-885, 2002.

Efforts to boost host NK cell-mediated immunity against solid tumors through IL-2 administration or the adoptive infusion of LAK cells have been largely unsuccessful. Atkins, et al., Med. Oncol., 18: 197-207, 2001; Rosenberg, et al., J. Natl. Cancer Inst., 85: 622-632, 1993. KIR-mediated inhibition of NK cell function by tumor HLAclass 1 or class 1-like ligands can be a major factor limiting autologous-based NK cell therapies. Murine and human studies have shown that alloreactive NK cells do not induce a GVH reaction. Ruggeri, et al., Science, 295: 2097-2100, 2002. These findings, combined with our data showing that KIR-incompatible NK cells have enhanced tumor cytotoxicity compared with KIR-matched or autologous NK populations, could be used as the rationale to investigate their antineoplastic potential in humans. Using irradiated EBV-LCLs as feeders, bulk NK populations were expanded 10⁴-fold, 10²-fold higher than the maximum expansion previously reported achievable when PBMCs were used as feeders. Therefore, it appears feasible that 10¹⁰ or more allogeneic NK cells could be expanded ex vivo for adoptive infusion from a starting source of only 10 to 25 mL of peripheral blood. Such populations would likely have to be irradiated before their adoptive infusion in the allogeneic setting to prevent long-term engraftment and possible transfusion-associated GVHD as a consequence of T-lymphocyte contamination. Not surprisingly, irradiation induced NK cell apoptosis and, ultimately, cell death. However, our in vitro data showed that some level ofNK cell cytotoxic function was maintained for up to 48 hours after exposure to 50 Gy. Indeed, γ-irradiated, partially mismatched allogeneic PBMC infusions have recently been reported to induce disease regression in some patients with metastatic RCC.39 Our data suggest that adoptively infused and irradiated allogeneic NK cells with KIR incompatibility could have similar or even enhanced antitumor effects compared with unmanipulated allogeneic PBMC infusions containing a relatively small proportion ofNK cells.

Preliminary data on fresh and early-passage tumor cells isolated from large metastatic lesions have shown similarly enhanced susceptibility to KIR-incompatible NK cells as was observed with established tumor lines.

Finally, allogeneic hematopoietic stem cell transplantation has recently been shown to induce graft-versus-tumor effects in some patients with advanced cytokine-refractory metastatic RCC. Storb, et al., Hematology (Am. Soc. Hematol. Educ. Program), 372-397,2003. Because HLA-matched siblings were used as donors, alloreactive NK cells would not be expected to expand. Data in this study showing enhanced susceptibility of RCC and melanoma cells to NK cells with KIR incompatibility suggest the NK cell-mediated antitumor effects seen against AML after KIR-incompatible mismatched allotransplantation might be induced in a similar fashion against select solid tumors. Transplantation trials for metastatic RCC using unrelated donors are already in progress. Ueno, et al., Blood, 102: 3829-3836, 2003. Based on our findings, trials that evaluate the use of single HLA-mismatched unrelated donors with KIR incompatibility (in the direction of the GVH) should be considered. This would also provide an opportunity to investigate the impact of posttransplantation alloreactive NK cell infusions, which have been previously shown to facilitate engraftment, reduce the risk for GVHD, and eradicate leukemia in murine models of mismatched transplantation. Ruggeri, et al., Science, 295: 2097-2100, 2002.

### EXAMPLE 8

### Method to Sensitize Tumor Cells to NK Cell Killing

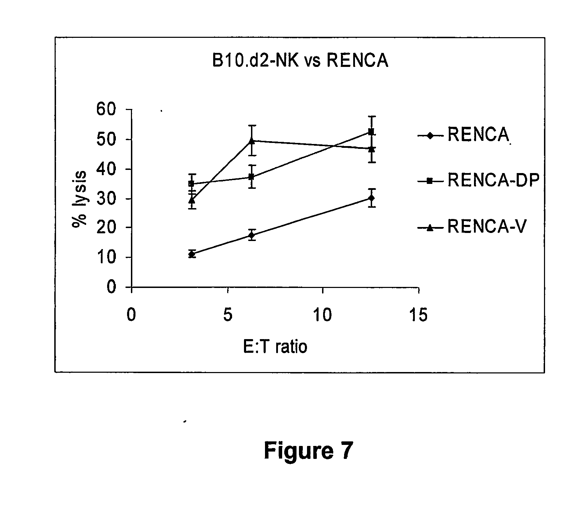

The present invention provides a method for treating a neoplastic disease by making tumor cells more susceptible to NK cell mediated lysis. The method provides treating the tumor cell with a potentiating agent, e.g., a chemical agent or chemotherapeutic agent and a composition comprising an enriched allogeneic NK cell population or a composition comprising an enriched autologous NK cell population. In Figure 7, RENCA tumor cells were treated with a chemical agent, e.g., Depsipeptide or Velcade, followed by an NK cell susceptibility assay. The results demonstrate that treating the tumor cells with the chemical agent in combination with an enriched NK cell population makes the tumor cells more susceptible to NK cell mediated lysis compared to treatment of the tumor cells with NK cells alone. One chemical agent, Depsipeptide, is an histone deacetylase inhibitor which interferes with the regulation of gene expression by inhibiting the enzymatic activity of histone deacetylase. Depsipeptide has been shown to have anti-tumor effects by inhibiting cell growth and preventing the formation of tumors in mice models. Experiments have shown that induction of MIC-A/B upon treatment with Depsipeptide. The chemical agent, Velcade (bortezamid), is a proteasome inhibitor. Experiments have shown that the proteasome inhibitor, PS-341, sensitizes neoplastic cells to TRAIL-mediated apoptosis by reducing levels of c-FLIP. Sayers TJ, Blood 102: 303-310, 2003. Further experiments have shown that the proteasome inhibitor, bortezomib, inhibits acute graft-versus-host disease while retaining graft-versus-tumor effects of the drug. Kai Sun, Proc. Nat. Acad. Sci. USA, 101: 8120-8125, 2004.

Figure 7 shows pretreatment of tumor cells with Depsipeptide or Velcade makes the tumor cells more susceptible to NK cell mediated lysis. RENCA tumor cells were treated for 40 hours with Depsipeptide [25 ng/ml] or Velcade [10 nM] and labeled with ⁵¹Cr for one hour in 37°C. Thereafter, the tumor cells were tested for NK cell susceptibility in a standard cytotoxicity assay. NK cells were purified from splenocytes from a B10.d2 mouse by negative magnetic bead depletion of T cells, B cells, monocytes and granulocytes. NK cell purity was evaluated by DX5 and CD3 staining by flow cytometry. Purity of NK cells was always between 90-100%.

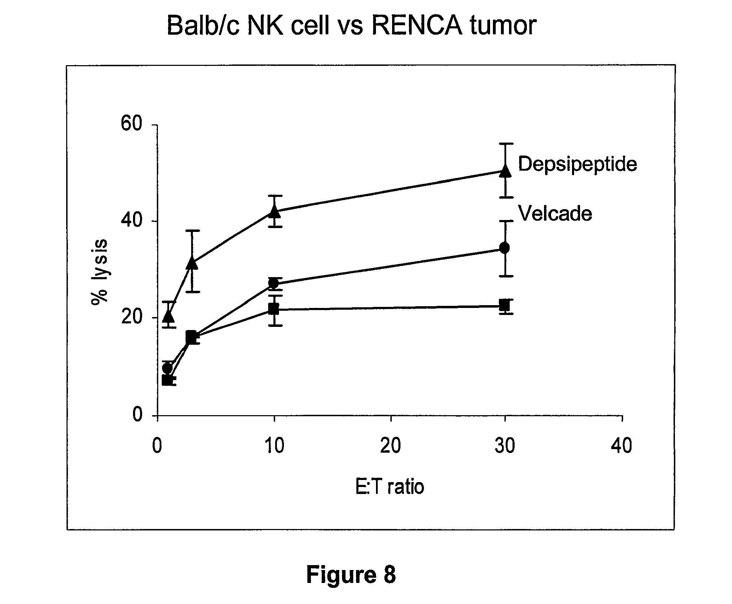

Figure 8 shows enhanced NK cell susceptibility of murine renal cell carcinoma (RENCA) pre-sensitized with Velcade or Depsipeptide. RENCA tumor cells were cultured in presence of 10nM Velcade or 25 ng/ml Depsipeptide for 14 hours. Cells were thereafter labeled with ⁵¹Cr for 1 hour and tested for susceptibility to NK cell mediated lysis in a standard 4 hour assay. Murine autologous Balb/c NK cells tested against untreated (squares), Velcade (circles) or Depsipeptide (triangles) treated RENCA cell lines (Balb/c in origin).

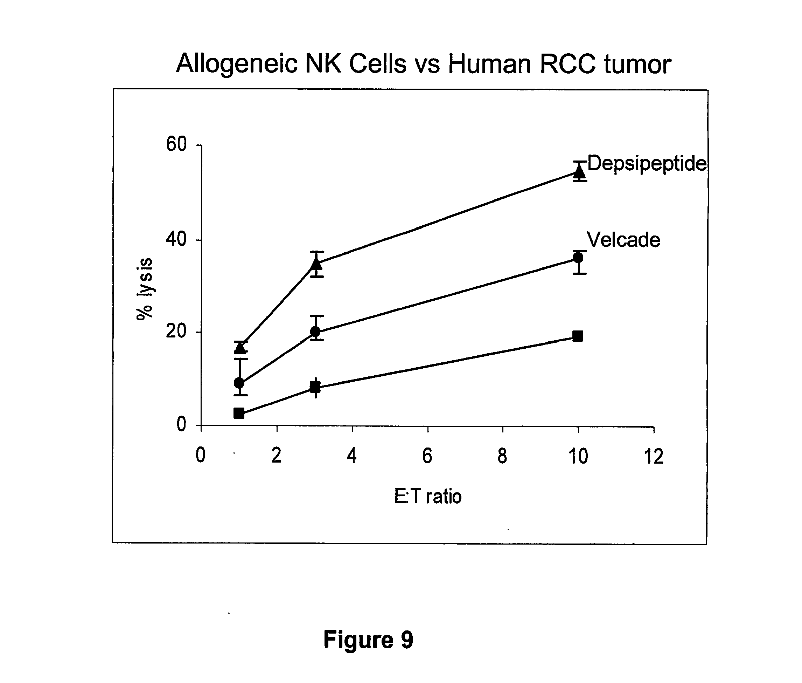

Figure 9 shows enhanced allogeneic NK cell susceptibility of human renal cell carcinoma (RCC) pre-sensitized with Velcade or Depsipeptide. Human (JOHW) and murine (RENCA) tumor cells were cultured in presence of 10nM Velcade or 25ng/ml Depsipeptide for 40 and 14 hours, respectively. Cells were thereafter labeled with ⁵¹Cr for 1 hour and tested for susceptibility to allogeneic NK cell mediated lysis in a standard 4 hour assay.

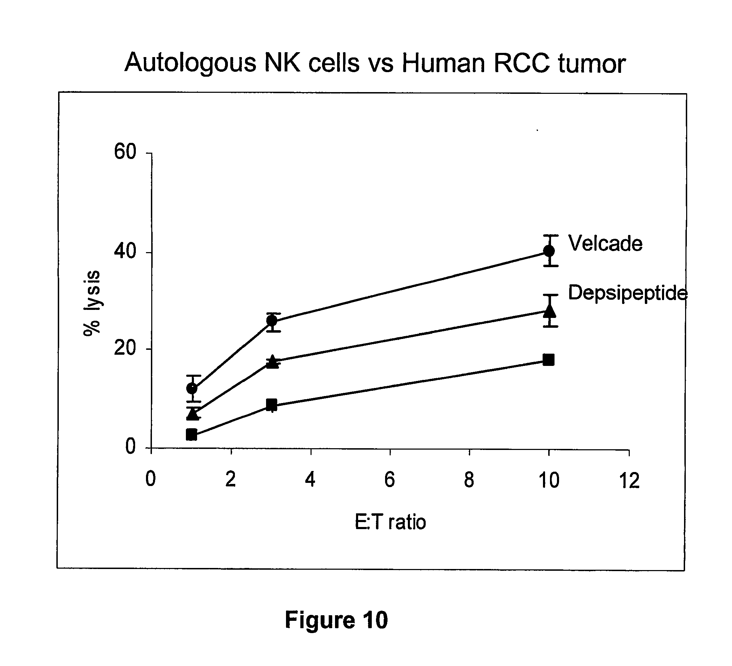

Figure 10 shows enhanced autologous NK cell susceptibility of human renal cell carcinoma (RCC) pre-sensitized with Velcade or Depsipeptide. Human (JOHW) and murine (RENCA) tumor cells were cultured in presence of 10 nM Velcade or 25 ng/ml Depsipeptide for 40 and 14 hours, respectively. Cells were thereafter labeled with ⁵¹Cr for 1 hour and tested for susceptibility to autologous NK cell mediated lysis in a standard 4 hour assay.

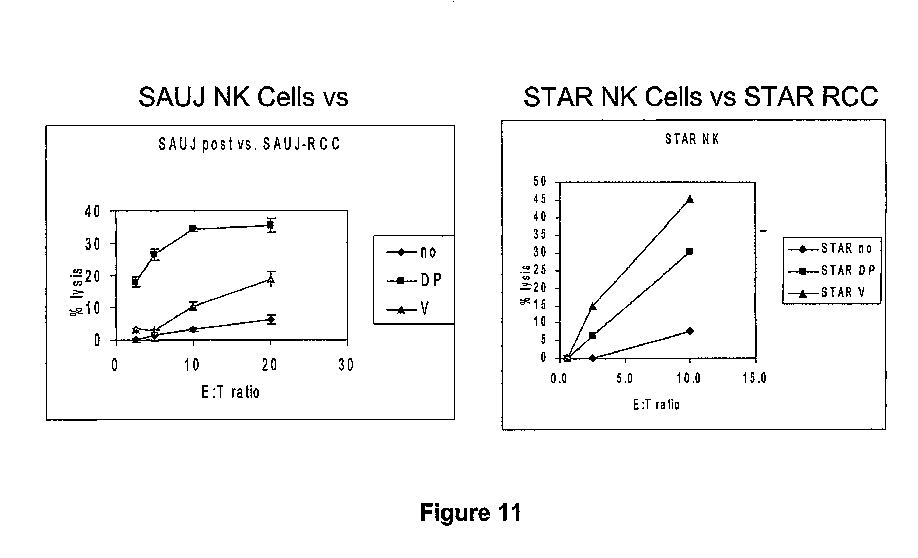

Figure 11 shows enhanced autologous NK cell susceptibility of human renal cell carcinoma (SAUJ RCC or STAR RCC) pre-sensitized with Velcade or Depsipeptide. Human RCC cell lines STAR or SAUJ were treated for 24 hours with 25 ng/ml Depsipeptide or 10 nM Velcade and labeled with ⁵¹Cr for one hour in 37°C. Thereafter, the tumor cells were tested for NK cell susceptibility in a standard cytotoxicity assay. Autologous NK cells were purified from peripheral blood by negative magnetic bead depletion of T cells, B cells, monocytes and granulocytes. NK cell purity was evaluated by CD56 and CD3 staining by flow cytometry. Purity of NK cells was always between 90-100%.

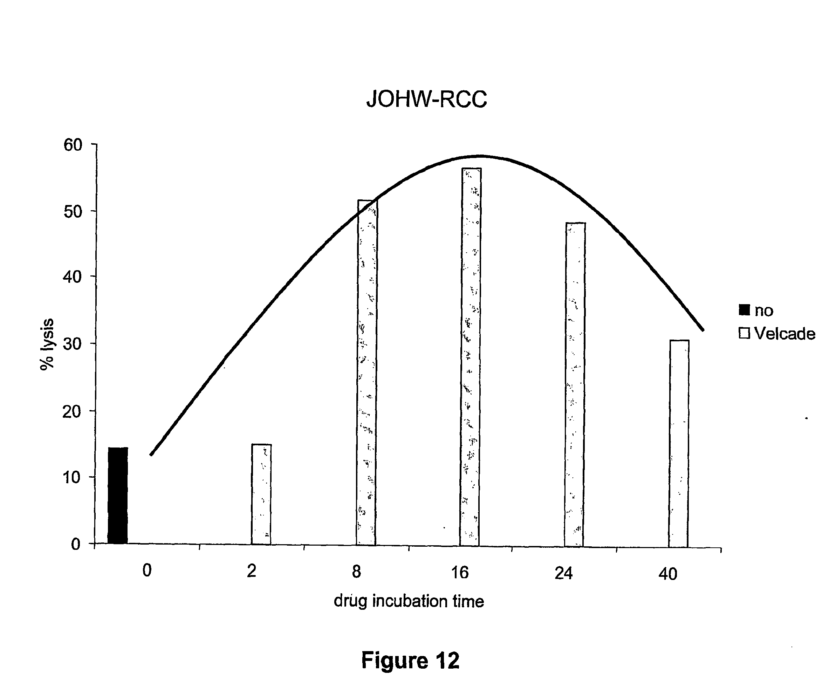

Figure 12 shows a time course to optimize chemical agent incubation or chemotherapeutic drug incubation with human RCC cell line. The human RCC cell line JOHW was treated with 10nM of Velcade for 2, 8, 16, 24 and 40 hours and thereafter labeled with hour for one hour in 37°C. The tumor cells were tested for NK cell susceptibility in a standard cytotoxicity assay. NK cells were purified from an allogeneic random donor by negative magnetic bead depletion of T cells (CD3) and positive magnetic bead selection of CD56⁺ cells. NK cell purity was evaluated by CD56 and CD3 staining by flow cytometry. Purity of NK cells was always between 90-100%.

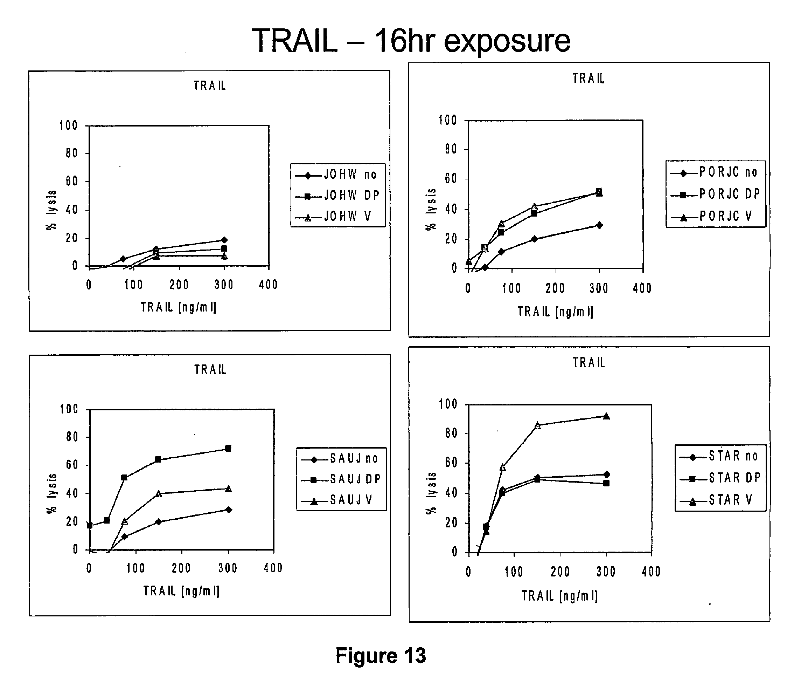

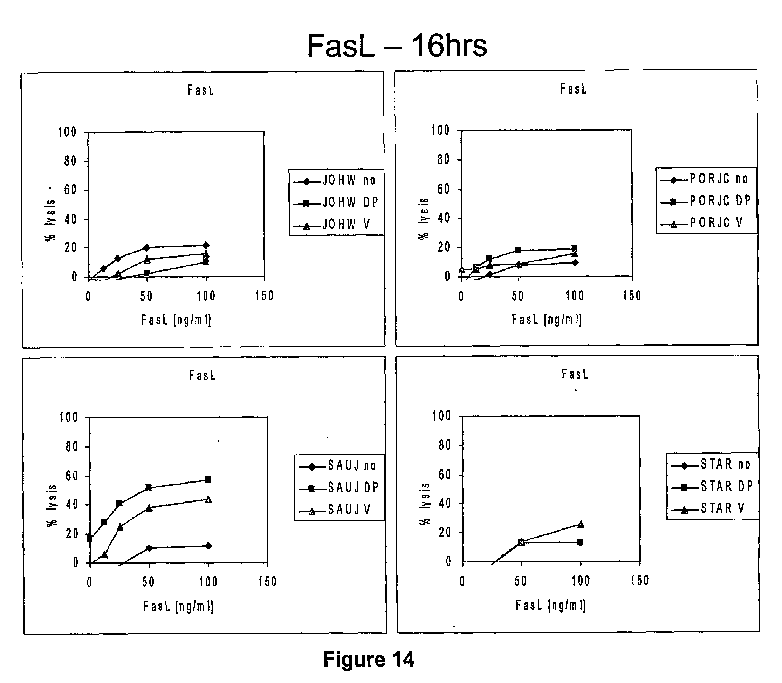

Enhanced NK cell susceptibility of human renal cell carcinoma (RCC) presensitized with Velcade or Depsipeptide may occur via a mechanism involving TRAIL (Figure 13) or Fas ligand (Figure 14). Increased cell lysis occurred in RCC cells presensitized with Velcade or Depsipeptide and following 16 hour incurbation with TRAIL or Fas ligand. Four human RCC cell lines (JOHW, PORJC, SAUJ and STAR) were treated with Depsipeptide [25 ng/ml] or Velcade [10 nM] for 24 hours and thereafter labeled with ⁵¹Cr for one hour in 37°C. Cell were then plated in a 96-well plate at 4,000 cell/well. Recombinant TRAIL [75-300 ng/ml] or recombinant Fas-ligand [12.5-100 ng/ml] was added to the cells and supernatant was harvested after 16 hours incubation. The level of lysis by TRAIL and Fas-ligand were calculated based on spontaneous and maximum ⁵¹Cr release.

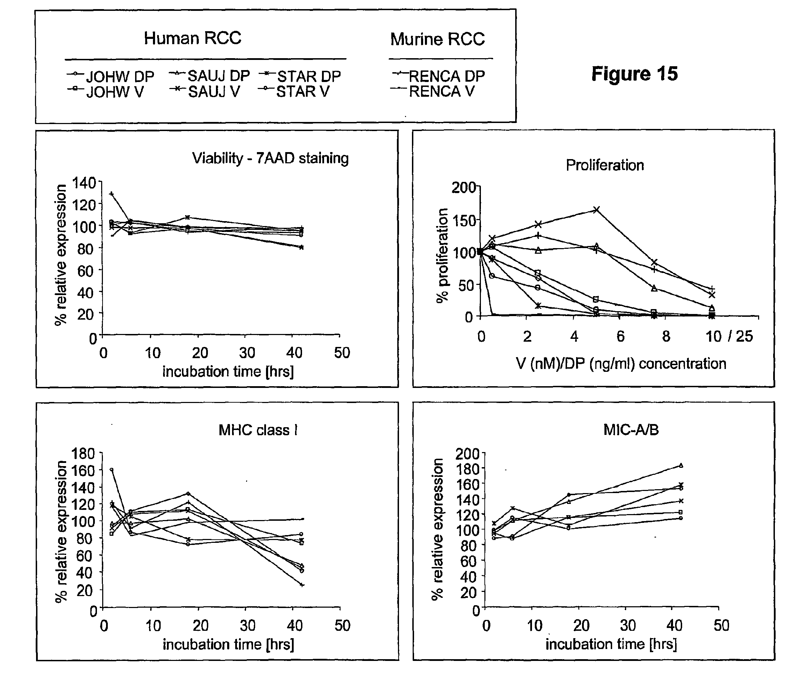

Figure 15 compares RCC cell lines and shows no differences in viability (apoptosis), proliferation, MHC Class I expression, or MIC-A/B expression. Three human RCC cell line (JOHW, SAUJ and STAR) and one murine RCC cell line (RENCA) were treated with Depsipeptide [25 ng/ml] or Velcade [10 nM] for 6-40 hours. Level of apoptosis was evaluated by Annexin-V and 7-AAD staining (upper left chart). Expression of MHC class I was evaluated by flow cytometry by HLA-ABC for human RCC and H-2Dd/Kd for murine RCC (lower left chart). MIC-A/B expression on human RCC was evaluated by monoclonal antibody staining and flow cytometry (lower right chart). Both human and murine RCC was treated at various concentration of Depsipeptide and Velcade for 72 hours. Cells were labeled with ³H thymidine for analysis of proliferation (upper right chart).

Further studies will measure potentiating agent, e.g., a chemical agent or chemotherapeutic agent sensitization to NK cell killing in other tumor cells, *e.g.*, prostate, colon, melanoma, lymphoma, or breast. Mechanism of action will be defined using micro-array analysis of the tumor and NK cells, blocking, or tumor phenotype. These assays will be used to predict responsiveness to therapy. A murine treatment model will be developed.

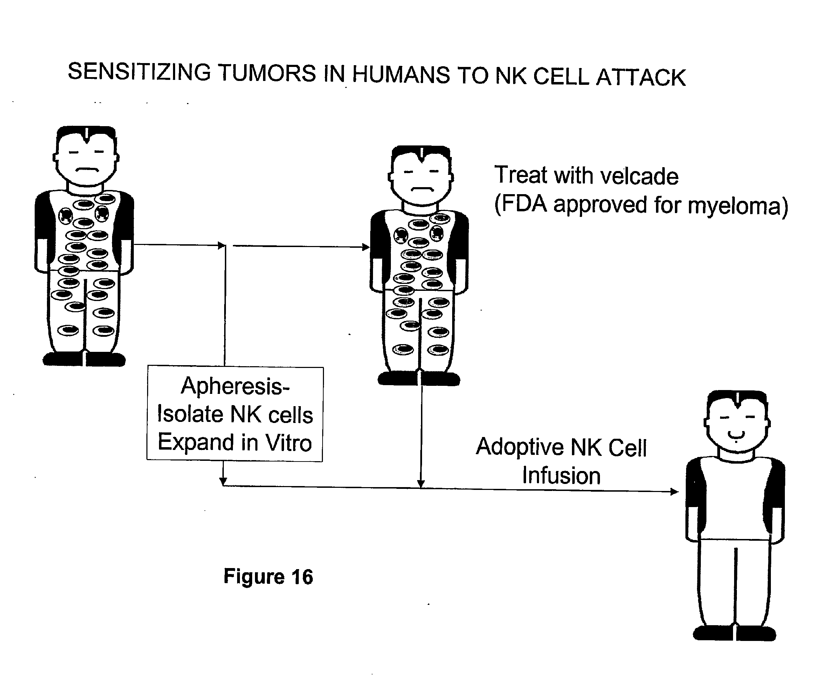

Figure 16 proposes a human clinical trial to sensitize tumors in humans to NK cell attack. NK cells will be isolated from a cancer patient by apheresis. NK cells will be expanded *in vitro.* The patient will be treated with a chemical agent, for example, Velcade (bortezomib), which is FDA approved for treatment of multiple myeloma, to sensitize to NK cell killing followed by an adoptive NK cell infusion.

When ranges are used herein for physical properties, such as molecular weight, or chemical properties, such as chemical formulae, all combinations and subcombinations of ranges and specific embodiments therein are intended to be included.

## Claims

1. The use of a potentiating agent for the manufacture of a medicament for treating a solid tumor wherein said treatment comprises administering the medicament and a composition comprising an enriched allogenic NK cell population, wherein the potentiating agent is a proteasome inhibitor or a histone deacetylase inhibitor and the potentiating agent sensitizes the tumor to killing by the NK cells.

2. The use of claim 1 wherein the potentiating agent is bortezomib.

3. The use of claim 1 wherein the potentiating agent is depsipeptide.

4. The use of a potentiating agent for the manufacture of a medicament for treating a solid tumor wherein the treatment comprises administering the medicament and a composition comprising an enriched autologous NK cell population, wherein the potentiating agent is a proteasome inhibitor or a histone deacetylase inhibitor and the potentiating agent sensitizes the tumor to killing by the NK cells.

5. The use of claim 4 wherein the potentiating agent is bortezomib.

6. The use of claim 4 wherein the potentiating agent is depsipeptide.

7. A potentiating agent for use in a method of treating a solid tumor wherein the treatment comprises administering the potentiating agent and a composition comprising an enriched allogenic NK cell population, wherein the potentiating agent is a proteasome inhibitor or a histone deacetylase inhibitor and the potentiating agent sensitizes the tumor to killing by the NK cells.

8. The potentiating agent of claim 7 wherein the potentiating agent is bortezomib.

9. The potentiating agent of claim 7 wherein the potentiating agent is depsipeptide.

10. A potentiating agent for use in a method of treating a solid tumor wherein the treatment comprises administering the potentiating agent and a composition comprising an enriched autologous NK cell population, wherein the potentiating agent is a proteasome inhibitor or a histone deacetylase inhibitor and the potentiating agent sensitizes the tumor to killing by the NK cells.

11. The potentiating agent of claim 10 wherein the potentiating agent is bortezomib.

12. The potentiating agent of claim 10 wherein the potentiating agent is depsipeptide.

13. A composition comprising an enriched allogenic NK cell population for use in a method of treating a solid tumor wherein the treatment comprises administering a potentiating agent and said composition, wherein the potentiating agent is a proteasome inhibitor or a histone deacetylase inhibitor and the potentiating agent sensitizes the tumor to killing by the NK cells.

14. The composition of claim 13 wherein the potentiating agent is bortezomib.

15. The composition of claim 13 wherein the potentiating agent is depsipeptide.

16. A composition comprising an enriched autologous NK cell population for use in a method of treating a solid tumor wherein the treatment comprises administering a potentiating agent and said composition, wherein the potentiating agent is a proteasome inhibitor or a histone deacetylase inhibitor and the potentiating agent sensitizes the tumor to killing by the NK cells.

17. The composition of claim 16 wherein the potentiating agent is bortezomib.

18. The composition of claim 16 wherein the potentiating agent is depsipeptide.

## Patentansprüche

1. Verwendung eines Potenzierungsmittels zur Herstellung eines Arzneimittels für die Behandlung eines festen Tumors, wobei die Behandlung die Verabreichung des Arzneimittels und einer Zusammensetzung, die eine angereicherte allogene NK-Zellpopulation umfasst, umfasst, wobei es sich bei dem Potenzierungsmittel um einen Proteasomeninhibitor oder einen Histondeacetylaseinhibitor handelt und das Potenzierungsmittel den Tumor für Abtötung durch die NK-Zellen sensibilisiert.

2. Verwendung nach Anspruch 1, wobei es sich bei dem Potenzierungsmittel um Bortezomib handelt.

3. Verwendung nach Anspruch 1, wobei es sich bei dem Potenzierungsmittel um Depsipeptid handelt.

4. Verwendung eines Potenzierungsmittels zur Herstellung eines Arzneimittels für die Behandlung eines festen Tumors, wobei die Behandlung die Verabreichung des Arzneimittels und einer Zusammensetzung, die eine angereicherte autologe NK-Zellpopulation umfasst, umfasst, wobei es sich bei dem Potenzierungsmittel um einen Proteasomeninhibitor oder einen Histondeacetylaseinhibitor handelt und das Potenzierungsmittel den Tumor für Abtötung durch die NK-Zellen sensibilisiert.

5. Verwendung nach Anspruch 4, wobei es sich bei dem Potenzierungsmittel um Bortezomib handelt.

6. Verwendung nach Anspruch 4, wobei es sich bei dem Potenzierungsmittel um Depsipeptid handelt.

7. Potenzierungsmittel zur Verwendung in einem Verfahren zur Behandlung eines festen Tumors, wobei die Behandlung die Verabreichung des Potenzierungsmittels und einer Zusammensetzung, die eine angereicherte allogene NK-Zellpopulation umfasst, umfasst, wobei es sich bei dem Potenzierungsmittel um einen Proteasomeninhibitor oder einen Histondeacetylaseinhibitor handelt und das Potenzierungsmittel den Tumor für Abtötung durch die NK-Zellen sensibilisiert.

8. Potenzierungsmittel nach Anspruch 7, wobei es sich bei dem Potenzierungsmittel um Bortezomib handelt.

9. Potenzierungsmittel nach Anspruch 7, wobei es sich bei dem Potenzierungsmittel um Depsipeptid handelt.

10. Potenzierungsmittel zur Verwendung in einem Verfahren zur Behandlung eines festen Tumors, wobei die Behandlung die Verabreichung des Potenzierungsmittels und einer Zusammensetzung, die eine angereicherte autologe NK-Zellpopulation umfasst, umfasst, wobei es sich bei dem Potenzierungsmittel um einen Proteasomeninhibitor oder einen Histondeacetylaseinhibitor handelt und das Potenzierungsmittel den Tumor für Abtötung durch die NK-Zellen sensibilisiert.

11. Potenzierungsmittel nach Anspruch 10, wobei es sich bei dem Potenzierungsmittel um Bortezomib handelt.

12. Potenzierungsmittel nach Anspruch 10, wobei es sich bei dem Potenzierungsmittel um Depsipeptid handelt.

13. Zusammensetzung, umfassend eine angereicherte allogene NK-Zellpopulation zur Verwendung in einem Verfahren zur Behandlung eines festen Tumors, wobei die Behandlung die Verabreichung eines Potenzierungsmittels und der Zusammensetzung umfasst, wobei es sich bei dem Potenzierungsmittel um einen Proteasomeninhibitor oder einen Histondeacetylaseinhibitor handelt und das Potenzierungsmittel den Tumor für Abtötung durch die NK-Zellen sensibilisiert.

14. Zusammensetzung nach Anspruch 13, wobei es sich bei dem Potenzierungsmittel um Bortezomib handelt.

15. Zusammensetzung nach Anspruch 13, wobei es sich bei dem Potenzierungsmittel um Depsipeptid handelt.

16. Zusammensetzung, umfassend eine angereicherte autologe NK-Zellpopulation zur Verwendung in einem Verfahren zur Behandlung eines festen Tumors, wobei die Behandlung die Verabreichung eines Potenzierungsmittels und der Zusammensetzung umfasst, wobei es sich bei dem Potenzierungsmittel um einen Proteasomeninhibitor oder einen Histondeacetylaseinhibitor handelt und das Potenzierungsmittel den Tumor für Abtötung durch die NK-Zellen sensibilisiert.

17. Zusammensetzung nach Anspruch 16, wobei es sich bei dem Potenzierungsmittel um Bortezomib handelt.

18. Zusammensetzung nach Anspruch 16, wobei es sich bei dem Potenzierungsmittel um Depsipeptid handelt.

## Revendications

1. Utilisation d'un agent potentialisateur pour la fabrication d'un médicament pour traiter une tumeur solide, ledit traitement comprenant l'administration du médicament et d'une composition comprenant une population de cellules NK allogéniques enrichie, l'agent potentialisateur étant un inhibiteur de protéasome ou un inhibiteur d'histone désacétylase et l'agent potentialisateur sensibilisant la tumeur à l'élimination par les cellules NK.

2. Utilisation de la revendication 1 dans laquelle l'agent potentialisateur est le bortézomib.

3. Utilisation de la revendication 1 dans laquelle l'agent potentialisateur est un dépsipeptide.

4. Utilisation d'un agent potentialisateur pour la fabrication d'un médicament pour traiter une tumeur solide dans laquelle le traitement comprend l'administration du médicament et d'une composition comprenant une population de cellules NK autologues enrichie, l'agent potentialisateur étant un inhibiteur de protéasome ou un inhibiteur d'histone désacétylase et l'agent potentialisateur sensibilisant la tumeur à l'élimination par les cellules NK.

5. Utilisation de la revendication 4 dans laquelle l'agent potentialisateur est le bortézomib.

6. Utilisation de la revendication 4 dans laquelle l'agent potentialisateur est un dépsipeptide.

7. Agent potentialisateur pour utilisation dans un procédé de traitement d'une tumeur solide, le traitement comprenant l'administration de l'agent potentialisateur et d'une composition comprenant une population de cellules NK allogéniques enrichie, l'agent potentialisateur étant un inhibiteur de protéasome ou un inhibiteur d'histone désacétylase et l'agent potentialisateur sensibilisant la tumeur à l'élimination par les cellules NK.

8. Agent potentialisateur de la revendication 7, l'agent potentialisateur étant le bortézomib.

9. Agent potentialisateur de la revendication 7, l'agent potentialisateur étant un dépsipeptide.

10. Agent potentialisateur pour utilisation dans un procédé de traitement d'une tumeur solide, le traitement comprenant l'administration de l'agent potentialisateur et d'une composition comprenant une population de cellules NK autologues enrichie, l'agent potentialisateur étant un inhibiteur de protéasome ou un inhibiteur d'histone désacétylase et l'agent potentialisateur sensibilisant la tumeur à l'élimination par les cellules NK.

11. Agent potentialisateur de la revendication 10, l'agent potentialisateur étant le bortézomib.

12. Agent potentialisateur de la revendication 10, l'agent potentialisateur étant un dépsipeptide.

13. Composition comprenant une population de cellules NK allogéniques enrichie pour utilisation dans un procédé de traitement d'une tumeur solide, le traitement comprenant l'administration d'un agent potentialisateur et de ladite composition, l'agent potentialisateur étant un inhibiteur de protéasome ou un inhibiteur d'histone désacétylase et l'agent potentialisateur sensibilisant la tumeur à l'élimination par les cellules NK.

14. Composition de la revendication 13, l'agent potentialisateur étant le bortézomib.

15. Composition de la revendication 13, l'agent potentialisateur étant un dépsipeptide.

16. Composition comprenant une population de cellules NK autologues enrichie pour utilisation dans un procédé de traitement d'une tumeur solide, le traitement comprenant l'administration d'un agent potentialisateur et de ladite composition, l'agent potentialisateur étant un inhibiteur de protéasome ou un inhibiteur d'histone désacétylase et l'agent potentialisateur sensibilisant la tumeur à l'élimination par les cellules NK.

17. Composition de la revendication 16, l'agent potentialisateur étant le bortézomib.

18. Composition de la revendication 16, l'agent potentialisateur étant un dépsipeptide.
